# EUROPEAN PATENT APPLICATION

(11) **EP 4 159 852 A1**
(43) Date of publication of application: **05.04.2023**
(21) Application number: 21818719.3
(22) Date of filing: 02.06.2021
(51) Int. Cl.: C12N 9/16, C12N 9/04, C12Q 1/28, C12Q 1/44, C12Q 1/60, G01N 33/92

(54) **KIT AND METHOD**

(30) Priority: 02.06.2020 JP 2020096095
(71) Applicant: Denka Company Limited, Tokyo 103-8338 (JP)
(72) Inventor: ITOH Yasuki, Tokyo 103-8338 (JP)
(74) Representative: Schnappauf, Georg
(86) International application number: PCT/JP2021/020962
(87) International publication number: WO 2021/246435

(57) **Abstract**

There is provided a kit that is used for fractionation of cholesterol (lipoprotein C) in a lipoprotein other than small dense LDL in a sample, the kit containing a first reagent composition having at least one activity selected from the group consisting of a cholesterol esterase activity and a cholesterol oxidase activity and a second reagent composition for quantifying the lipoprotein C of a measurement target, where a ratio R1 represented by ABS400/ABS450 is 0.90 or more and 3.50 or less, and in an absorption spectrum after storing the second reagent composition at 37°C for two weeks, the ratio R1 represented by ABS400/ABS450 is 0.90 or more and 9.00 or less.

## Description

### TECHNICAL FIELD

The present invention relates to a kit and a method.

### BACKGROUND ART

Cholesterol is one of the major constitutional components of a cell. Excess cholesterol is a clinically important component since it is incorporated by macrophages under the endothelial cells of blood vessels to form foam cells and exhibits early lesions of arteriosclerosis. Cholesterol is transported in the blood in the form of a lipoprotein. The lipoprotein includes very low density lipoprotein (VLDL), high density lipoprotein (HDL), low density lipoprotein (LDL), and the like, which have functions different from each other. Here, various lipoproteins are further classified into subfractions. As the lipoprotein subfractions, HDL is classified into HDL3, which has a smaller particle size and higher density, and HDL2, which has a larger particle size and a lower density. HDL can be divided into apoE containing HDL and apoE deficient HDL based on the difference in the content of apolipoprotein E (apoE). Regarding VLDL, there is a remnant reduced in size with respect to the normal size, which is formed by degradation by a lipoprotein lipase.

Cholesterol in the lipoprotein including these subfractions can be measured using a general-purpose automated analysis device.

Specifically, techniques for measuring cholesterol in the lipoprotein are described in Patent Documents 1 to 5. In these Patent Documents, absorbance measurement at a wavelength of about 600 to 700 nm on a long wavelength side has been used at the time of quantification of cholesterol in the lipoprotein.

### RELATED DOCUMENT

### PATENT DOCUMENTS

[Patent Document 1] International Publication No. WO98/026090
[Patent Document 2] Japanese Unexamined Patent Publication No. H10-038888
[Patent Document 3] International Publication No. WO2012/011554
[Patent Document 4] Japanese Unexamined Patent Publication No. 2012-100581
[Patent Document 5] Japanese Unexamined Patent Publication No. 2013-215169

### SUMMARY OF THE INVENTION

### TECHNICAL PROBLEM

As a result of examining the above-described technique for quantifying cholesterol in the lipoprotein, the inventor of the present invention revealed that there is still room for improvement in terms of stably obtaining a high level of measurement accuracy.

The present invention provides a technique for stably quantifying cholesterol in lipoprotein (lipoprotein C) with high accuracy.

### SOLUTION TO PROBLEM

The inventor of the present invention examined the quantification of lipoprotein C using absorbance measurement in a wavelength range of about 600 to 700 nm so as to obtain high measurement accuracy even for a trace amount of sample. As a result, a new problem was found that there is a case where a reagent that is used for quantification deteriorates when stored, which causes absorption having a peak on a wavelength side shorter than the wavelength range in the quantification of the lipoprotein C, and the influence of such absorption is observed over the high wavelength side. It has also revealed that the occurrence of such absorption affects the absorbance at the time of the quantification of the lipoprotein C, which may reduce the measurement accuracy. In addition, it was also found that how the absorption peak appears on the short wavelength side may vary depending on, for example, the components of the reagent that is used for measurement, the storage conditions, and the number of storage days.

Therefore, as a result of further examinations to remove the influence of the absorption occurring in the short wavelength region as much as possible, it was newly found that in a case where in a reagent composition that is used for fractionation of lipoprotein C, an absorbance ratio after an accelerated test is set to a specific range at a specific wavelength outside the measurement wavelength region for lipoprotein C, the influence of the absorption on the long wavelength side can be effectively suppressed, and it is possible to quantify lipoprotein C with high accuracy, whereby the present invention was completed.

According to the present invention, the following kits and methods are provided.
[1] A kit that is used for fractionation of cholesterol (lipoprotein C) in a lipoprotein other than small dense LDL in a sample, the kit containing:
   a first reagent composition having at least one activity selected from the group consisting of a cholesterol esterase activity and a cholesterol oxidase activity; and
   a second reagent composition for quantifying the lipoprotein C as a measurement target,
   in which in an absorption spectrum after storing the first reagent composition at 37°C for two weeks, a ratio R1 represented by ABS400/ABS450 is 0.90 or more and 3.50 or less in a case where an absorbance at a wavelength of 400 nm is denoted by ABS400 and an absorbance at a wavelength of 450 nm is denoted by ABS450, and
   in an absorption spectrum after storing the second reagent composition at 37°C for two weeks, a ratio R1 represented by ABS400/ABS450 is 0.90 or more and 9.00 or less in a case where an absorbance at a wavelength of 400 nm is denoted by ABS400 and an absorbance at a wavelength of 450 nm is denoted by ABS450.
[2] The kit according to [1], in which in the absorption spectrum after storing the first reagent composition at 37°C for 2 weeks, a ratio R2 represented by ABS360/ABS400 is 0.90 or more and 3.00 or less in a case where an absorbance at a wavelength of 360 nm is denoted by ABS360.
[3] The kit according to [1] or [2], in which the first reagent composition has at least one activity selected from the group consisting of a peroxidase activity and a catalase activity.
[4] The kit according to any one of [1] to [3], in which the first reagent composition contains a surfactant that acts on a lipoprotein other than the lipoprotein as a measurement target.
[5] The kit according to any one of [1] to [4], in which in the absorption spectrum after storing the second reagent composition at 37°C for 2 weeks, a ratio R2 represented by ABS360/ABS400 is 0.90 or more and 2.50 or less in a case where an absorbance at a wavelength of 360 nm is denoted by ABS360.
[6] The kit according to any one of [1] to [5], in which the second reagent composition contains a surfactant that acts on the lipoprotein as a measurement target.
[7] The kit according to any one of [1] to [6], in which the first reagent composition satisfies one or two of the following Conditions 1 to 3, and
   the second reagent composition does not satisfy the one or two of Conditions 1 to 3 but satisfies all conditions other than the one or two of Conditions 1 to 3:
   Condition 1: containing a coupler;
   Condition 2: containing an iron complex; and
   Condition 3: having peroxidase activity.
[8] The kit according to any one of [1] to [7], in which the lipoprotein C as a measurement target is LDL cholesterol, HDL cholesterol, HDL3 cholesterol, remnant cholesterol, or apoE containing HDL cholesterol.
[9] A method of quantifying cholesterol (lipoprotein C) in a lipoprotein other than small dense LDL in a sample, the method including:
   causing a first reagent composition having at least one activity selected from the group consisting of a cholesterol esterase activity and a cholesterol oxidase activity, to act on the sample; and
   after the causing the first reagent composition to act on the sample, causing the second reagent composition for quantifying the lipoprotein C as a measurement target to act, to quantify cholesterol in a remaining lipoprotein,
   in which in an absorption spectrum after storing the first reagent composition at 37°C for two weeks, a ratio R1 represented by ABS400/ABS450 is 0.90 or more and 3.50 or less in a case where an absorbance at a wavelength of 400 nm is denoted by ABS400 and an absorbance at a wavelength of 450 nm is denoted by ABS450, and
   in an absorption spectrum after storing the second reagent composition at 37°C for two weeks, a ratio R1 represented by ABS400/ABS450 is 0.90 or more and 9.00 or less in a case where an absorbance at a wavelength of 400 nm is denoted by ABS400 and an absorbance at a wavelength of 450 nm is denoted by ABS450.
[10] The method according to [9], in which in the absorption spectrum after storing the first reagent composition at 37°C for 2 weeks, a ratio R2 represented by ABS360/ABS400 is 0.90 or more and 3.00 or less in a case where an absorbance at a wavelength of 360 nm is denoted by ABS360.
[11] The method according to [9] or [10], in which the first reagent composition further has at least one activity selected from the group consisting of a peroxidase activity and a catalase activity.
[12] The method according to any one of [9] to [11], in which the first reagent composition contains a surfactant that acts on a lipoprotein other than the lipoprotein as a measurement target.
[13] The method according to any one of [9] to [12], in which in the absorption spectrum after storing the second reagent composition at 37°C for 2 weeks, a ratio R2 represented by ABS360/ABS400 is 0.90 or more and 2.50 or less in a case where an absorbance at a wavelength of 360 nm is denoted by ABS360.
[14] The method according to any one of [9] to [13], in which the second reagent composition contains a surfactant that acts on the lipoprotein as a measurement target.
[15] The method according to any one of [9] to [14], in which the first reagent composition satisfies one or two of the following Conditions 1 to 3, and the second reagent composition does not satisfy the one or two of Conditions 1 to 3 but satisfies all conditions other than the one or two of Conditions 1 to 3:
   Condition 1: containing a coupler;
   Condition 2: containing an iron complex; and
   Condition 3: having peroxidase activity.
[16] The method according to any one of [9] to [15], in which the lipoprotein C as a measurement target is LDL cholesterol, HDL cholesterol, HDL3 cholesterol, remnant cholesterol, or apoE containing HDL cholesterol.

### ADVANTAGEOUS EFFECTS OF INVENTION

According to the present invention, it is possible to provide a technique for stably quantifying cholesterol in lipoprotein with high accuracy.

### BRIEF DESCRIPTION OF THE DRAWINGS

Fig. 1 is a graph showing absorbance measurement results of first and second reagent compositions.
Fig. 2 is a graph showing absorbance measurement results of first and second reagent compositions.
Fig. 3 is a graph showing absorbance measurement results of first and second reagent compositions.
Fig. 4 is a graph showing absorbance measurement results of first and second reagent compositions.
Fig. 5 is a graph showing absorbance measurement results of first and second reagent compositions.
Fig. 6 is a graph showing absorbance measurement results of first and second reagent compositions.
Fig. 7 is a graph showing absorbance measurement results of first and second reagent compositions.
Fig. 8 is a graph showing absorbance measurement results of first and second reagent compositions.
Fig. 9 is a graph showing absorbance measurement results of first and second reagent compositions.
Fig. 10 is a graph showing absorbance measurement results of first and second reagent compositions.
Fig. 11 is a graph showing absorbance measurement results of first and second reagent compositions.

### DESCRIPTION OF EMBODIMENTS

Hereinafter, embodiments will be described. In this embodiment, a composition such as a measurement reagent can contain respective components alone or in a combination of two or more thereof. In the present specification, a numerical range "x to y" indicates "x or more and y or less" and includes both the lower limit value x and the upper limit value y.

First, lipoprotein and lipoprotein C are described.

The lipoprotein is broadly divided into a chylomicron (CM), VLDL, LDL, and HDL, and respective lipoproteins are further divided into subfractions. Some of these fractions and subfractions can be distinguished by particle size or specific gravity. Although the diameter of the particle size thereof varies depending on the reporter, VLDL has a diameter of 30 nm to 80 nm (30 nm to 75 nm), LDL including intermediate density lipoprotein (IDL) has a diameter of 22 nm to 28 nm (19 nm to 30 nm), HDL has a diameter of 7 to 12 nm, HDL3 which is an HDL subfraction has a diameter of 7 to 8.5 nm, and HDL2 has a diameter of 8.5 to 10 nm. VLDL has a specific gravity of 1.006 or less, IDL has a specific gravity of 1.006 to 1.019, LDL has a specific gravity of 1.019 to 1.063, HDL has a specific gravity of 1.063 to 1.21, HDL2 has a specific gravity of 1.063 to 1.125, and HDL3 has a specific gravity of 1.125 to 1.210. It is possible to measure the particle diameter according to, for example, gradient gel electrophoresis (GGE) (JAMA, 260, p. 1917-21, 1988), NMR (HANDBOOK OF LIPOPROTEIN TESTING, 2nd Edition, edited by Nader Rifai et al., AACC PRESS: The Fats of Life Summer 2002, LVDD 15 YEAR ANNIVERSARY ISSUE, Volume AVI No.3, p. 15-16), and it is possible to determine the specific gravity according to, for example, analysis by ultracentrifugation (Atherosclerosis, 106, p. 241-253, 1994: Atherosclerosis, 83, p. 59, 1990).

It is possible to measure HDL cholesterol (HDL-C) and LDL cholesterol (LDL-C), for example, with an automated analysis device.

In addition, HDL can be classified into apoE containing HDL and apoE deficient HDL subfractions based on the difference in the content rate of ApoE. Generally, apoE containing HDL indicates one that contains ApoE in HDL, and apoE deficient HDL indicates one that does not contain ApoE in HDL. HDL in which ApoE is present or the content ApoE is large is sometimes called apoE rich HDL, which is also included in the ApoE containing HDL. Since the distribution of the content of ApoE present inside HDL is continuous, it is not possible to clearly distinguish between apoE containing HDL and apoE deficient HDL by the ApoE content ratio in the lipoprotein. It is possible to quantify apoE containing HDL-C, for example, by subtracting a measured value from the 13% PEG method (J Lipid Research 38, 1204-16: 1997) that can measure the total HDL-C including apoE containing HDL-C from a measured value from the phosphotungstic acid-dextran sulfate-magnesium precipitation method (the PT-DS-Mg method) (BIOCHEMICAL MEDICINE AND METABOLIC BIOLOGY 46, 329-343 (1991)) that enables the measurement of only apoE deficient HDL-C without measuring apoE containing HDL-C. In addition, apoE containing HDL-C can be measured with an automated analysis device by using the difference in reactivity depending on the surfactant concentration (Ann Clin Biochem 56, 123-132 (2019)).

The remnant is a special lipoprotein that is generated in a case where lipid metabolism is stagnant due to metabolic syndrome or the like, and it is a metabolite generated from CM or VLDL through the degradation by the action of a lipoprotein lipase. Although the remnant is not defined by a specific size or specific gravity, it can be quantified by using a gel in which an antibody against a constitutional apolipoprotein in the lipoprotein is immobilized, a phospholipid-degrading enzyme, a surfactant, or the like. In addition, the remnant-cholesterol (the remnant-C) can be measured with an automated analysis device by using the difference in reactivity depending on the molecular weight of the cholesterol esterase (J Applied Laboratory Medicine 3, 26-36, (2018)).

This embodiment specifically relates to the fractionation or measurement of cholesterol (lipoprotein C) in a lipoprotein other than small dense LDL (sdLDL-C). sdLDL generally refers to a subfraction of the LDL fraction, which has a diameter of about 22.0 to about 25.5 nm or a specific gravity of 1.040 to 1.063. The range of the specific gravity of sdLDL varies slightly depending on the reporter, and in another report, sdLDL is a fraction in a range of 1.044 to 1.060 (Atherosclerosis: 106 241-253 1994).

In the present specification, in a case where sdLDL is referred to, it specifically refers to an LDL that has a higher specific gravity among LDLs and is clinically more atherogenic than other LDLs. In addition, sdLDL preferably refers to an LDL having a specific gravity belonging to a range higher than the central point in the specific gravity range of LDLs and more preferably having a specific gravity belonging to a range of 1.044 to 1.063.

In addition, a lipoprotein other than sdLDL is referred to, it specifically refers to a chylomicron, VLDL, IDL, LDL having a specific gravity outside a range of 1.044 to 1.063, HDL, or a subfraction thereof.

In this embodiment, the lipoprotein C as a measurement target is, for example, LDL cholesterol (LDL-C), HDL cholesterol (HDL-C), HDL3 cholesterol (HDL3-C), remnant cholesterol (remnant-C), or apoE containing HDL cholesterol (apoE containing HDL-C).

### (Kit)

In this embodiment, the kit is used for fractionation of cholesterol in a lipoprotein (lipoprotein C) other than the small dense LDL (sdLDL-C) in a sample, and it contains the following first reagent composition and second reagent composition.

### (First reagent composition)

The first reagent composition is a reagent composition having at least one activity selected from the group consisting of a cholesterol esterase activity and a cholesterol oxidase activity. In an absorption spectrum after storing the first reagent composition at 37°C for two weeks, a ratio R1 represented by ABS400/ABS450 is 0.9 or more and 3.50 or less in a case where an absorbance at a wavelength of 400 nm is denoted by ABS400 and an absorbance at a wavelength of 450 nm is denoted by ABS450.

### (Second reagent composition)

The second reagent composition is a reagent composition for quantifying the lipoprotein C as a measurement target. In an absorption spectrum after storing the second reagent composition at 37°C for two weeks, a ratio R1 represented by ABS400/ABS450 is 0.90 or more and 9.00 or less in a case where an absorbance at a wavelength of 400 nm is denoted by ABS400 and an absorbance at a wavelength of 450 nm is denoted by ABS450.

The inventor of the present invention found that in a case where an absorbance ratio of each of the first and second reagent compositions after an accelerated test is set to a specific range at a specific wavelength outside the measurement wavelength region for lipoprotein C, it is possible to quantify lipoprotein C with high accuracy. Specifically, it was found that the ratio R1 of ABS400/ABS450 is suitable as an index of the presence or absence and the degree of the absorption on the short wavelength side. By setting R1 equal to or smaller than the upper limit value, buffering to the measurement wavelength range of sdLDL-C, for example, an absorption wavelength region of 600 to 700 nm, from an adsorption having a peak on the lower wavelength side than the wavelength region of 600 to 700 nm can be effectively suppressed. Thus, it is possible to quantify lipoprotein C with high accuracy.

Here, although it has been difficult to determine the presence or absence of buffering in the measurement wavelength region for the lipoprotein C itself, by using absorbances at 400 nm and 450 nm outside the observation region and an absorbance ratio of each of the first and second reagent compositions after an accelerated test as an index, it is possible to grasp the presence of the absorption having a peak on the short wavelength side and the effect of buffering. In addition, it is possible to improve measurement accuracy by using a reagent composition in which R1 is within a specific range.

In the absorption spectrum of the first reagent composition after being stored at 37°C for two weeks, in a case where an absorbance at a wavelength of 400 nm is denoted by ABS400 and an absorbance at a wavelength of 450 nm is denoted by ABS450, the ratio R1 represented by ABS400/ABS450 is 3.50 or less, preferably 3.25 or less, more preferably 3.00 or less, still more preferably 2.80 or less, even still more preferably 2.60 or less, and even further still more preferably 2.50 or less, from the viewpoint of improving the accuracy of the lipoprotein C measurement.

In addition, from the viewpoint of stably quantifying the lipoprotein C, R1 of the first reagent composition is 0.90 or more, and it may be, for example, 0.95 or more or 1.00 or more.

In addition, in the absorption spectrum of the second reagent composition after being stored at 37°C for two weeks, in a case where an absorbance at a wavelength of 400 nm is denoted by ABS400 and an absorbance at a wavelength of 450 nm is denoted by ABS450, the ratio R1 represented by ABS400/ABS450 is 9.00 or less, preferably 8.00 or less, more preferably 6.00 or less, still more preferably 4.00 or less, even more preferably 3.00 or less, even still more preferably 2.50 or less, and even further still more preferably 2.00 or less, from the viewpoint of improving the accuracy of the lipoprotein C measurement.

In addition, from the viewpoint of stably quantifying the lipoprotein C, R1 of the second reagent composition is 0.90 or more, and it may be, for example, 0.95 or more or 1.00 or more.

In this embodiment, since both the first and second reagent compositions contained in the kit satisfy the above conditions for R1, lipoprotein C can be measured with high accuracy.

In the absorption spectrum of the first reagent composition after being stored at 37°C for two weeks, in a case where an absorbance at a wavelength of 360 nm is denoted by ABS360, the ratio R2 represented by ABS360/ABS400 is, for example, 3.00 or less, and it is preferably 2.50 or less, more preferably 2.20 or less, still more preferably 2.00 or less, even still more preferably 1.68 or less, and even further still more preferably 1.60 or less, from the viewpoint of more stably improving the accuracy of the lipoprotein C measurement.

In addition, from the viewpoint of still more stably quantifying the lipoprotein C, R2 of the first reagent composition may be, for example, 0.90 or more, and it may be, for example, 0.95 or more or 1.00 or more.

In addition, in the absorption spectrum of the second reagent composition after being stored at 37°C for two weeks, in a case where an absorbance at a wavelength of 360 nm is denoted by ABS360, the ratio R2 represented by ABS360/ABS400 is, for example, 3.00 or less, and it is preferably 2.50 or less, more preferably 2.00 or less, still more preferably 1.70 or less, and even still more preferably 1.50 or less, from the viewpoint of more stably improving the accuracy of the lipoprotein C measurement.

In addition, from the viewpoint of still more stably quantifying the lipoprotein C, R2 of the second reagent composition may be, for example, 0.90 or more, and it may be, for example, 0.95 or more or 1.00 or more.

From the viewpoint of further improving the accuracy of the lipoprotein C measurement, both the first and second reagent compositions more preferably satisfy the above conditions for R2.

In this embodiment, the kit is used for the fractionation or quantification of the lipoprotein C and more preferably used for the fractionation and quantification of the lipoprotein C.

In addition, the kit is specifically used for a quantification method for lipoprotein C, which includes two or more steps. In a case of being used, the first and second reagent compositions are each used in a different step, and they are preferably used in the order of the first and second reagent compositions.

The configuration of each reagent composition will be described in more detail below.

### (First reagent composition)

The first reagent composition has at least one activity selected from the group consisting of a cholesterol esterase activity and a cholesterol oxidase activity and preferably has a cholesterol esterase activity and a cholesterol oxidase activity.

Since the first reagent composition is a composition having the above-described one or two or more activities, it is possible to erase, for example, a lipoprotein other than the lipoprotein as a measurement target in a sample in a case where the first reagent composition is added to the sample. In addition, the cholesterol in a lipoprotein other than the lipoprotein as a measurement target can be led out of the reaction system.

Here, "a surfactant acts (reacts)" refers to that a surfactant degrades a lipoprotein to liberate cholesterol in the lipoprotein. For example, in a case of being referred to as "a surfactant that acts on (reacts with) a lipoprotein other than the lipoprotein as a measurement target", it is not required that the surfactant does not act on the lipoprotein as a measurement target at all, and it suffices that the surfactant mainly acts on a lipoprotein other than the lipoprotein as a measurement target. "Erasing" means degrading a substance in a test sample so that a degradation product thereof is not detected in the next step. That is, "erasing cholesterol in a lipoprotein other than the lipoprotein as a measurement target" means that a lipoprotein other than the lipoprotein as a measurement target in a test sample is degraded so that the cholesterol in the lipoprotein, which is a degradation product of the lipoprotein, is not detected in the subsequent step.

"Leading out of the reaction system" refers to erasing or aggregating cholesterol contained in a lipoprotein not to be measured or inhibiting it so that a reaction does not occur in the subsequent step so that the cholesterol contained in the lipoproteins not to be measured does not affect the quantification of the lipoprotein C as a measurement target.

In addition, "has a cholesterol esterase activity" specifically refers to that a cholesterol esterase is present and a reaction catalyzed by cholesterol esterase can occur. The same applies to other enzymatic activities such as a cholesterol oxidase activity, a sphingomyelinase activity, a catalase activity, and a peroxidase activity.

Next, the component contained in the first reagent composition will be described more specifically.

Specific examples of the component contained in the first reagent composition include an enzyme, a protein having no enzymatic action, a surfactant, a buffer solution, a salt, a hydrogen donor, a coupler, and an iron complex.

### (Enzyme)

The first reagent composition is, for example, a composition containing one or two or more enzymes, having at least one activity selected from the group consisting of a cholesterol esterase activity and a cholesterol oxidase activity, and it preferably contains an enzyme having a cholesterol esterase activity and an enzyme having a cholesterol oxidase activity.

In addition, the first reagent composition is, for example, a composition containing at least one enzyme selected from the group consisting of a cholesterol esterase and a cholesterol oxidase, and it preferably contains a cholesterol esterase and a cholesterol oxidase.

As the cholesterol esterase and the cholesterol oxidase, it is possible to use, for example, those derived from bacteria or fungi.

From the viewpoint of stably leading cholesterol in a lipoprotein other than the lipoprotein as a measurement target out of the reaction system, the cholesterol esterase activity of the first reagent composition is preferably 10 U/L or more, more preferably 300 U/L or more, still more preferably 600 U/L or more, and it is preferably 10,000 U/L or less, more preferably 2,500 U/mL or less, and still more preferably 2,000 U/L or less.

From the same viewpoint as above, the cholesterol oxidase activity of the first reagent composition is preferably 100 U/L or more, more preferably 200 U/L or more, still more preferably 300 U/m or more, and it is preferably 3,500 U/L or less, more preferably 3,000 U/L or less, and still more preferably 2,500 U/L or less.

The first reagent composition may have an activity other than the above activity. From the viewpoint of more stably leading cholesterol in a lipoprotein other than the lipoprotein as a measurement target in a sample out of the reaction system, it has, for example, at least one activity selected from the group consisting of a catalase activity, a peroxidase activity, and a sphingomyelinase activity and preferably has at least one activity selected from the group consisting of a peroxidase activity and a catalase activity.

More specifically, the first reagent composition may contain an enzyme having an activity other than the activity described above. For example, the first reagent composition further contains an enzyme having an activity other than the activity described above. It preferably contains an enzyme having at least one activity selected from the group consisting of a catalase activity, a peroxidase activity, and a sphingomyelinase activity and more preferably contains an enzyme having at least one activity selected from the group consisting of a catalase activity and a peroxidase activity.

In addition, the first reagent composition contains, for example, at least one enzyme selected from the group consisting of a catalase, a peroxidase, and a sphingomyelinase and preferably contains at least one of a catalase and a peroxidase.

From the viewpoint of more stably leading cholesterol in a lipoprotein other than the lipoprotein as a measurement target in a sample out of the reaction system, the catalase activity of the first reagent composition is preferably 40 U/mL or more and more preferably 100 U/mL or more, and it is preferably 2,500 U/mL or less and more preferably 2,000 U/mL or less.

In a case where the first reagent composition has a catalase activity, it is also preferable that the lipoprotein C as a measurement target is HDL-C, LDL-C, HDL3-C, remnant-C, or apoE containing HDL-C.

From the viewpoint of more stably leading cholesterol in a lipoprotein other than the lipoprotein as a measurement target in a sample out of the reaction system, the peroxidase activity of the first reagent composition is preferably 400 U/L or more and more preferably 600 U/L or more, and it is preferably 3,000 U/L or less and more preferably 2,000 U/L or less.

From the viewpoint of more stably leading cholesterol in a lipoprotein other than the lipoprotein as a measurement target in a sample out of the reaction system, the sphingomyelinase activity of the first reagent composition is preferably 100 U/L or more and more preferably 200 U/L or more, and it is preferably 100,000 U/L or less and more preferably 20,000 U/L or less.

It is also preferable that the lipoprotein C as a measurement target is HDL3-C or remnant-C in a case where the first reagent composition has a sphingomyelinase activity.

On the other hand, in a case where the lipoprotein as a measurement target is LDL other than sdLDL, the first reagent composition preferably does not have a sphingomyelinase activity.

Specific examples of the sphingomyelinase include SPC (manufactured by Asahi Kasei Corporation), Sphingomyelinase from bacillus cereus, and Sphingomyelinase from staphylococcus aureus (manufactured by Sigma-Aldrich Co. LLC).

In addition, the first reagent composition may preferably contain a lipoprotein degrading enzyme from the viewpoint of preferably adjusting the action on various lipoproteins.

As the lipoprotein degrading enzyme, it is possible to use, for example, a lipoprotein lipase. The lipoprotein lipase is not limited as long as it is an enzyme capable of degrading a lipoprotein, and it is possible to use, for example, a lipoprotein lipase derived from an animal or a microorganism.

In a case where the first reagent composition contains a lipoprotein lipase, the lipoprotein lipase activity of the first reagent composition is preferably 100 U/L or more, and it is preferably 10,000 U/L or less, more preferably 5,000 U/L or less, and still more preferably 1,000 U/L or less, from the viewpoint of preferably adjusting the action on various lipoproteins.

Here, a method of measuring the enzymatic activity of the first reagent composition and the second reagent composition described below will be described.

In this embodiment, each of the cholesterol esterase activity, the cholesterol oxidase activity, the catalase activity, the peroxidase activity, the sphingomyelinase activity, and the lipoprotein lipase activity of the reagent composition can be measured, for example, by the following method.

In the measurement of the cholesterol esterase activity, a substrate (a solution of 0.04% cholesterol linolenic acid, 1% Triton X100, and 0.6% sodium cholate), a solution of 300 U/mL cholesterol oxidase, an enzyme dilution solution (a 20 mM phosphate buffer solution containing 0.5 mM EDTA·2Na, 2 mM MgCl₂, and 0.2% bovine serum albumin (BSA), pH 7.5), and a reaction solution (0.06% 4-aminoantipyrine, 0.4% phenol, 7.5 KU/L peroxidase) are used. After mixing 1.75 mL of the reaction solution and 1.0 mL of the substrate solution, the resultant mixture is heated at 37°C for 5 minutes, and 0.1 mL of the cholesterol oxidase solution is added thereto. After heating at 37°C for 2 minutes, 0.1 mL of a measurement target diluted with the dilution solution is added thereto, the resultant mixed solution is allowed to react at 37°C, and the amount of change in an absorbance at a wavelength of 500 nm is measured. After the reaction at 37°C, the amount of change in the absorbance from 0 to 3.5 minutes is measured to calculate the cholesterol esterase activity. For example, in a case where the amount of change in absorbance is 8 U/L or more, it can be said that the measurement target has a cholesterol esterase activity, and more specifically, it can be said that the measurement target contains a cholesterol esterase.

In the measurement of the cholesterol oxidase activity, a 6 mM cholesterol solution (the cholesterol is in isopropanol) is used as a substrate solution. A dilution solution (a 0.1 M phosphate buffer solution containing Triton X100, pH 7.0) is added so that the concentration of a measurement target is 2 to 4 U/mL, 3 mL of a diluted solution is heated at 37°C for 5 minutes and then added to 0.05 mL of the substrate solution. Then, the mixed solution is allowed to react at 37°C, and the amount of change in the absorbance at a wavelength of 240 nm is measured. After the reaction at 37°C, the amount of change in absorbance is measured from 2 minutes to 7 minutes, and the cholesterol oxidase activity is calculated. For example, in a case where the amount of change in absorbance is 3 U/L or more, it can be said that the measurement target has a cholesterol oxidase activity, and more specifically, it can be said that the measurement target contains a cholesterol oxidase.

In the measurement of the catalase activity, a substrate (a 50 mM phosphate buffer solution containing 0.06% hydrogen peroxide, pH 7.0) is used. After preheating 2.9 mL of the substrate solution at 25°C, the substrate solution is mixed with 0.1 mL of a measurement target, and the amount of change in the absorbance at 240 nm is measured. After the reaction at 25°C, the amount of change in the absorbance from 0 to 3 minutes is measured to calculate the catalase activity. For example, in a case where the amount of change in absorbance is 100 U/L or more, it can be said that the measurement target has a catalase activity, and more specifically, it can be said that the measurement target contains a catalase.

In the measurement of the peroxidase activity, a reaction solution 1 (a 50 mM phosphate buffer solution containing 1.5 mM HDAOS and 0.05% Triton X100, pH 7.0) and a reaction solution 2 (a 50 mM phosphate buffer solution containing 5 mM 4-aminoantipyrine, 0.05% Triton X100, and 1% hydrogen peroxide, pH 7.0), and a dilution solution (a 50 mM phosphate buffer solution, pH 7.0) are used. 0.3 mL of the reaction solution 1 and 0.08 mL of a measurement target diluted with the dilution solution are mixed, and the resultant mixture is heated at 37°C for 5 minutes. Then, 0.1 mL of the reaction solution 2 is added thereto, the resultant mixture is allowed to react at 37°C, and the amounts of change in the absorbance at a main wavelength of 600 nm and a minor wavelength of 700 nm are measured. After the reaction at 37°C, the amount of change in the absorbance from 2 minutes to 5 minutes is measured to calculate the peroxidase activity. For example, in a case where the amount of change in absorbance is 10 U/L or more, it can be said that the measurement target has a peroxidase activity, and more specifically, it can be said that the measurement target contains a peroxidase.

In the measurement of the sphingomyelinase activity, a reaction solution (a mixed solution of 0.008% sphingomyelin, a 0.05% Triton X100 solution, 10 U/mL alkaline phosphatase, 10 U/mL cholesterol oxidase, 2 U/mL peroxidase, 0.02% 4-aminoantipyrine, and 0.02% TODB), a reaction stopping solution (a 1% sodium dodecyl sulfate solution), and a dilution solution (a 10 mM Tris buffer solution containing 0.1% Triton X100, pH 8.0) are used. 0.08 mL of the reaction solution and 0.003 mL of a measurement target diluted with the dilution solution are mixed, the resultant mixture is heated at 37°C for 5 minutes, and then 0.16 mL of the reaction stopping solution is added thereto. After stopping the reaction, the amount of change in the absorbance at a main wavelength of 546 nm and a minor wavelength of 700 nm is measured to calculate the sphingomyelinase activity. For example, in a case where the amount of change in absorbance is 2 U/L or more, it can be said that the measurement target has a sphingomyelinase activity, and more specifically, it can be said that the measurement target contains a sphingomyelinase.

The lipoprotein lipase activity of the first reagent composition is measured, for example, by the following method. That is, in the measurement of the lipoprotein lipase activity, an olive oil emulsion solution (a solution obtainable by adding 5 mL of 5.0% Triton X 100 to 5 g of olive oil and 2 mL of ethanol, subjecting the resultant mixture to sonication for 20 minutes, subsequently adding thereto 25 mL of 4% BSA and 15 mL of a 0.1 M phosphate buffer solution at pH 7.0, and carrying stirring at room temperature for 1 to 2 hours) is used as a substrate solution. A dilution solution (a 20 mM phosphate buffer solution containing 2 mM MgCl₂ and 0.5 mM EDTA-2Na, pH 7.5) is added to the substrate solution preheated at 37°C for 5 minutes so that the concentration is 0.9 to 1.6 U/ml, 0.2 mL of a diluted sample is added thereto, and after heating at 37°C for 15 minutes, 2.0 mL of a reaction stopping solution (0.2 M trichloroacetic acid) is added thereto. Then, filtration (ADVANTEC No. 131 manufactured by Toyo Roshi Kaisha, Ltd., or Whatman No. 42) is carried out to collect the filtrate. To 0.05 mL of the filtrate, 3.0 mL of a color developing reagent (200 mL of a 50 mM MES-NaOH buffer solution, 4 mL of 5% Triton X100, 40 µL of N,N-dimethyl-m-toluidine, 4 mg of 4-aminoantipyrine, 24.2 mg of ATP·2Na·3H₂O, 40.7 mg of MgCl₂·6H₂O, 200 U of glycerol kinase, 500 U of Lα glycerophosphate oxidase, 300 U of peroxidase) is added, the resultant mixed solution is allowed to react at 37°C for 15 minutes, the absorbance at a wavelength of 545 nm is measured, and the lipoprotein lipase activity is calculated. For example, in a case where the amount of change in absorbance is 3 U/L or more, it can be said that the measurement target has a lipoprotein lipase activity, and more specifically, it can be said that the measurement target contains a lipoprotein lipase.

In addition, enzymes in the first and second reagent compositions can also be identified by the following method. That is, first, fragment peptides obtained by digesting a sample containing a target enzyme with trypsin are detected with a hybrid type mass spectrometer. The mass of each peptide, which is obtained by the mass spectrometer, and fragment ion spectra (MS/MS data) obtained by causing peptides to collide with argon gas in a mass spectrometer are subjected to database searches (for example, the Mascot search), whereby proteins can be identified. In a case where the sequence of the fragment peptide derived from the amino acid sequence in the reagent composition uniquely matches with an amino acid sequence registered in the database, the target enzyme can be regarded as being contained in the reagent composition.

In addition, enzymes in the first and second reagent compositions can also be identified, for example, by the following quantification. That is, among fragment peptides obtainable by digesting a target enzyme with trypsin, a peptide that is specific to the target enzyme and gives a strong signal in mass spectrometry is selected as a peptide to be quantified. An unlabeled peptide and a peptide labeled with a stable isotope as an internal standard are produced by chemical synthesis for the peptide to be quantified. A sample containing the target enzyme is completely digested with trypsin, a known amount of the stable isotope labeled peptide is added, and the measurement is carried out with a triple quadrupole type mass spectrometer (LC-MS/MS) connected to HPLC, in a multiple reaction monitoring mode (an MRM mode). A mixed solution of the unlabeled peptide and the known amount of the stable isotope-labeled peptide of the peptide to be quantified is measured in the same way to create a calibration curve of the concentration ratio and peak area ratio of the internal standard, and the absolute amount of the peptide to be quantified in the sample is calculated, whereby the target enzyme can be quantified.

### (Protein having no enzymatic action)

Specific examples of the protein having no enzymatic action include albumin such as BSA.

From the viewpoint of stabilizing the enzyme in the first reagent composition and the viewpoint of leading the cholesterol in a lipoprotein other than the lipoprotein as a measurement target out of the reaction system, the BSA concentration in the first reagent composition is preferably 1 g/L or more and more preferably 2 g/L or more, and it is preferably 20 g/L or less and more preferably 10 g/L or less, with respect to the entire composition of the first reagent composition.

In a case where the first reagent composition contains BSA, it is also preferable that the lipoprotein C as a measurement target is LDL-C, HDL3-C, remnant-C, or apoE containing HDL-C.

### (Surfactant)

From the viewpoint of more stably leading cholesterol in a lipoprotein other than the lipoprotein as a measurement target in a sample out of the reaction system, the first reagent composition preferably contains a surfactant. The surfactant is specifically one or more selected from the group consisting of a nonionic surfactant, an anionic surfactant, a cationic surfactant, and an amphoteric surfactant, and it is preferably a nonionic surfactant and more preferably a polyoxyethylene derivative.

Examples of surfactants include a surfactant that acts on a lipoprotein other than the lipoprotein as a measurement target, and more preferred examples thereof include a surfactant that acts on a lipoprotein other than the lipoprotein as a measurement target but does not act on the lipoprotein as a measurement target. In addition, from the viewpoint of stably fractionating the lipoprotein as a measurement target, the first reagent composition preferably contains a surfactant that acts on a lipoprotein other than the lipoprotein as a measurement target and more preferably contains a surfactant that acts on a lipoprotein other than the lipoprotein as a measurement target but does not act on the lipoprotein as a measurement target.

For example, in a case where the lipoprotein as a measurement target is HDL or HDL3, the surfactant in the first reagent composition can be specifically one or more selected from the group consisting of a nonionic surfactant such as a polyoxyethylene sorbitan derivative, a polyoxyethylene-polyoxypropylene condensate, or a polyoxyethylene stearylamine; an anionic surfactant such as an amide ether sulfate and a sodium polyoxyethylene alkyl ether sulfate; an amphoteric surfactant such as a coconut oil fatty acid-amidopropyldimethyl-aminoacetic acid betaine, an alkyldimethyl-aminoacetic acid betaine, or a lauryl betaine; and a cationic surfactant such as lauryltrimethylammonium chloride.

In a case where the lipoprotein as a measurement target is HDL or HDL3, specific examples of the commercially available surfactant in the first reagent composition include, as nonionic surfactants, NONION OT-221, which is polyoxyethylene sorbitan monooleate (manufactured by NOF Corporation), Pluronic F68 (manufactured by ADEKA CORPORATION), Pluronic F88 (manufactured by ADEKA CORPORATION), Pluronic F127 (manufactured by ADEKA CORPORATION), Pluronic P103 (manufactured by ADEKA CORPORATION), and Pluronic P123 (manufactured by ADEKA CORPORATION), which are polyoxyethylene-polyoxypropylene condensates, NYMEEN S210 (manufactured by NOF Corporation) and EMULGEN A500 (manufactured by Kao Corporation), which are polyoxyethylene stearylamine; they include, as specific examples of the anionic surfactant, SUNAMID CF-10 (manufactured by NOF Corporation), which is an amide ether sulfate, and LEVENOL WX (manufactured by Kao Corporation), which is a sodium polyoxyethylene alkyl ether sulfate; they include, as specific examples of the amphoteric surfactants, NISSAN ANON (registered trade name) BDF-SF (manufactured by NOF Corporation), which is a coconut oil fatty acid-amidopropyldimethyl-aminoacetic acid betaine, NISSAN ANON (registered trade name) BF (manufactured by NOF Corporation), which is an alkyldimethyl-aminoacetic acid betaine, and AMPHITOL 24B (manufactured by Kao Corporation), which is a lauryl betaine; and they include, as specific examples of the cationic surfactant, QUARTAMIN 24P (manufactured by Kao Corporation), which is lauryltrimethylammonium chloride.

In a case where the lipoprotein as a measurement target is LDL or apoE containing HDL, examples of the surfactant that acts on a lipoprotein other than the lipoprotein as a measurement target include a nonionic surfactant, and specific examples thereof include a polyoxyethylene derivative. Examples of the polyoxyethylene derivative include one or two or more nonionic surfactants selected from the group consisting of polyoxyethylene alkyl ethers and polyoxyethylene polycyclic phenyl ethers.

Among the above, preferred examples of the polyoxyethylene polycyclic phenyl ether include a polyoxyethylene benzylphenyl derivative, a polyoxyethylene styrenated phenyl ether derivative, and a special phenol ethoxylate. Specific examples of the polyoxyethylene polycyclic phenyl ether include EMULGEN A-60, EMULGEN A-500, EMULGEN B-66, and EMULGEN A-90 (all, manufactured by Kao Corporation), Newcol 703, Newcol 704, and Newcol 706, Newcol 707, Newcol 708, Newcol 709, Newcol 710, Newcol 711, Newcol 712, Newcol 714, Newcol 719, Newcol 723, Newcol 729, Newcol 733, Newcol 740, Newcol 747, Newcol 780, Newcol 610, Newcol 2604, Newcol 2607, Newcol 2609, and Newcol 2614 (all, manufactured by Nippon Nyukazai Co., Ltd.), NOIGEN EA-87, NOIGEN EA-137, NOIGEN EA-157, NOIGEN EA-167, NOIGEN EA-177, NOIGEN EA-197D, and NOIGEN EA-207D (all, manufactured by DKS Co., Ltd.), and BLAUNON DSP-9, BLAUNON DSP-12.5, BLAUNON TSP-7.5, BLAUNON TSP-16, and BLAUNON TSP-50 (all, manufactured by AOKI OIL INDUSTRIAL Co., Ltd.).

Further, in a case where the lipoprotein as a measurement target is a remnant, examples of the surfactant that acts on a lipoprotein other than the lipoprotein as a measurement target include a polyoxyethylene derivative having an HLB value of 11 or more and 13 or less, and specific examples thereof include those having a desired HLB value among surfactants selected from the group consisting of a polyoxyethylene alkyl ether, a polyoxyalkylene alkyl ether, a polyoxyethylene alkyl phenyl ether, and a polyoxyethylene polycyclic phenyl ether. Examples of the commercially available product of such surfactants include EMULGEN A-60, EMULGEN 707, EMULGEN 709, and EMULGEN 909 (all, manufactured by Kao Corporation), and BLAUNON DSP-9 and BLAUNON DSP-12.5 (all, manufactured by AOKI OIL INDUSTRIAL Co., Ltd.).

From the viewpoint of causing a surfactant to stably act on a lipoprotein other than the lipoprotein as a measurement target, the concentration of the surfactant in the first reagent composition may be, for example, 0.02% (w/v) or more, is preferably 0.2% (w/v) or more, more preferably 0.3% (w/v) or more, and still more preferably 0.5% (w/v) or more, and it is preferably 5% (w/v) or less and more preferably 3% (w/v) or less, with respect to the entire composition of the first reagent composition.

In addition, in a case where the lipoprotein as a measurement target is apoE containing HDL, from the same viewpoint, the concentration of the surfactant in the first reagent composition is preferably 0.03 % (w/v) or more, more preferably 0.05% (w/v) or more, and it is preferably 1.2% (w/v) or less and more preferably 1.0% (w/v) or less, with respect to the entire composition of the first reagent composition.

It is noted that the surfactant contained in the first and second reagent compositions can be identified by a method of carrying out analysis by combining IR, NMR, LC-MS, and the like. Examples of the method of checking the ionicity (nonionicity, anionicity, or cationicity) of the surfactant include an extraction method with an organic solvent under acidic or alkaline conditions and a solid-phase extraction method. Examples of the method of determining the structure of the surfactant include a method of carrying out analysis by using LC-MSMS or NMR.

### (Buffer solution and salt)

The kind of buffer solution can be appropriately selected, for example, depending on the kind of enzyme contained in the first reagent composition. Specific examples of buffer solutions include a phosphate buffer solution, a Tris buffer solution, a PIPES (piperazine-1,4-bis(2-ethanesulfonic acid)) buffer solution, a BES (N,N-bis(2-hydroxyethyl)-2-aminoethanesulfonic acid) buffer solution.

From the viewpoint of maintaining the enzyme activity in the composition and the viewpoint of improving the storage stability of the reagent, the concentration of the buffer solution in the first reagent composition is preferably 1 mM or more, more preferably 5 mM or more, and still more preferably 10 mM or more, and it is preferably 300 mM or less, more preferably 200 mM or less, still more preferably 150 mM or less, and even still more preferably 100 mM or less.

A salt is blended specifically as a pH adjusting agent or an ionic strength adjusting agent. Specific examples of the salt include basic substances such as sodium salts such as sodium hydroxide and sodium sulfate; potassium salts such as potassium hydroxide; and ammonium salts such as ammonium sulfate and ammonium chloride. In addition, in a case where the first reagent composition contains at least one of a monovalent cation and a divalent cation, or a salt thereof, the fractionation of LDL in the measurement of the LDL other than sdLDL, for example, the fractionation of sdLDL and L LDL becomes easier.

From the viewpoint of causing a salt to act more stably as a pH adjusting agent or an ionic strength adjusting agent, the concentration of the salt in the first reagent composition is preferably 0.2 g/L or more and more preferably 0.5 g/L or more, and it is preferably 5 g/L or less and more preferably 3 g/L or less, with respect to the entire composition of the first reagent composition.

Further, in a case where the first reagent composition has a sphingomyelinase activity, a polyanion can be added in a first step in order to adjust the catalytic activity of sphingomyelinase with respect to the lipoprotein as a measurement target and another lipoprotein.

As the polyanion to be added, heparin, phosphotungstic acid, dextran sulfate, or the like can be suitably used.

The concentration of the polyanion in the first reagent composition is preferably 10 to 250 U/mL in a case of heparin, preferably 0.02% to 1.25% (w/v) in a case of phosphotungstic acid, and preferably 0.02% to 1.25% (w/v) in a case of dextran sulfate.

From the viewpoint of maintaining the enzymatic activity in the composition and the viewpoint of improving the storage stability of the reagent, the pH of the first reagent composition is preferably 6 or more and more preferably 6.5 or more, and it is preferably 8 or less and more preferably 7.5 or less.

### (Hydrogen donor and coupler)

The first reagent composition preferably contains any one of a hydrogen donor and a coupler. A hydrogen donor and a coupler are used in a combination in which a coupling reaction proceeds, for example, in the presence of a peroxidase activity. In addition, any one of the hydrogen donor and the coupler is used in the first step to lead the lipoprotein C other than the measurement target out of the reaction system. More specifically, any one of the hydrogen donor and the coupler is used in order to cause the cholesterol esterase or the cholesterol oxidase to act on the lipoprotein C other than the measurement target and convert the generated hydrogen peroxide to colorless quinone in the presence of the peroxidase activity.

Specific examples of the hydrogen donor include aniline derivatives such as N-ethyl-N-(2-hydroxy-3-sulfopropyl)-3-methylaniline (TOOS), N-ethyl-N-(2-hydroxy-3-sulfopropyl)-3,5-dimethylaniline (MAOS), N-ethyl-N-(3-sulfopropyl)-3-methylaniline (TOPS), N-(2-hydroxy-3-sulfopropyl)-3,5-dimethoxyaniline (HDAOS), N-(3-sulfopropyl)aniline (HALPS), N-(3-sulfopropyl)-3-methoxy-5-aniline (HMMPS), N-ethyl-N-(2-hydroxy-3-sulfopropyl)-4-fluoro-3,5-dimethoxyaniline (FDAOS), and N-ethyl-N-(3-methylphenyl)-N'-succinylethylenediamine (EMSE).

From the viewpoint of leading the lipoprotein C other than the measurement target out of the reaction system, the concentration of the hydrogen donor in the first reagent composition is preferably 1 mmol/L or more and more preferably 1.5 mmol/L or more, and it is preferably 5 mmol/L or less and more preferably 3 mmol/L or less.

In addition, in a case where the hydrogen donor is contained in the second reagent composition described later, the first reagent composition preferably contains a coupler that is used for the coupling reaction.

Specifically, the coupler is at least one selected from the group consisting of 4-aminoantipyrine, an aminoantipyrine derivative, vanillindiaminesulfonic acid, methylbenzthiazolinone hydrazone, and sulfonated methylbenzthiazolinone hydrazone.

From the viewpoint of leading the lipoprotein C other than the measurement target out of the reaction system, the concentration of the coupler in the first reagent composition is preferably 0.5 mM or more and more preferably 1.0 mM or more, and it is preferably 4 mM or less and more preferably 3 mM or less.

### (Iron complex)

Examples of the iron complex include those described below for the second reagent composition. The concentration of the iron complex is preferably 0.001 to 0.05 mmol/L.

The first reagent composition can have a configuration which includes, for example, an enzyme that degrades cholesterol, such as the cholesterol esterase or the cholesterol oxidase, any one of the hydrogen donor the coupler, and the catalase that erase hydrogen peroxide.

### (Second reagent composition)

The second reagent composition is a reagent for quantifying the lipoprotein C as a measurement target. Although the components of the second reagent composition differ depending on the configuration of the first reagent composition, any known substances may be used as long as they are to form a composition that allows the quantification of the lipoprotein C as a measurement target.

Examples of the components of the second reagent composition include an enzyme, a surfactant, a protein having no enzymatic action, a buffer solution, a salt, a coupler, a hydrogen donor, an iron complex, and a catalase inhibitor.

### (Enzyme)

The second reagent composition has, for example, a peroxidase activity. In addition, the second reagent composition contains, for example, an enzyme having a peroxidase activity and more specifically peroxidase.

From the viewpoint of accurately measuring the lipoprotein C as a measurement target, the peroxidase activity of the second reagent composition is preferably 500 U/L or more and more preferably 1,000 U/L or more, and it is preferably 20,000 U/L or less and more preferably 10,000 U/L or less.

However, since the peroxidase activity is carried over to a step of measuring the lipoprotein C as a measurement target (a second step described later) in a case where the first reagent composition has a peroxidase activity, it is also possible to reduce the concentration or make the peroxidase activity not contained in the second reagent.

### (Surfactant)

A surfactant can be appropriately selected depending on the lipoprotein as a measurement target.

Examples of the surfactant include a surfactant that acts on the lipoprotein as a measurement target. In addition, from the viewpoint of stably quantifying the lipoprotein C, the second reagent composition preferably contains a surfactant that acts on the lipoprotein as a measurement target.

The surfactant that acts on the lipoprotein as a measurement target may be a surfactant that selectively acts on the lipoprotein as a measurement target, such as a surfactant that acts only on the lipoprotein as a measurement target, and it may be a surfactant that also acts on a lipoprotein other than the lipoprotein as a measurement target or a surfactant that acts on all lipoproteins.

In a case where a lipoprotein other than the lipoprotein as a measurement target remains in the step of measuring the lipoprotein C as a measurement target, the second reagent composition preferably contains a surfactant that selectively acts on the lipoprotein as a measurement target.

For example, in a case where the lipoprotein as a measurement target is HDL or apoE containing HDL, the surfactant is, for example, one or more selected from the group consisting of polyoxyethylene lauryl ether, polyoxyethylene cetyl ether, polyoxyethylene oleyl ether, polyoxyethylene higher alcohol (having 4 to 35 carbon atoms), polyoxyethylene octyl phenyl ether, polyoxyethylene nonyl phenyl ether, and a polyoxyethylene polycyclic phenyl ether.

In a case where the lipoprotein as a measurement target is LDL or a remnant, the surfactant includes, for example, a polyoxyethylene derivative, and it is possible to use a surfactant that is used in a commercially available reagent for measuring total cholesterol or the like. Such a surfactant is, for example, one or more selected from the group consisting of a polyoxyethylene alkyl phenyl ether (for example, EMULGEN 909 (Kao Corporation) or Triton X100), a polyoxyethylene alkyl ether (for example, EMULGEN 707 and EMULGEN 709 (all, manufactured by Kao Corporation)), a polyoxyethylene-polyoxypropylene copolymer or a derivative thereof (for example, Pluronic (registered trade name)-type surfactant (BASF SE or ADEKA Corporation) such as Pluronic 17R-4, Pluronic L-64, Pluronic PE3100, Pluronic P-85, Pluronic F-88, Pluronic P-103, or Pluronic F-127), and a polyoxyethylene polycyclic phenyl ether (for example, EMULGEN B66, EMULGEN A90, and EMULGEN A60 (all, manufactured by Kao Corporation)).

In a case where the lipoprotein as a measurement target is HDL3, the surfactant is preferably a surfactant that acts selectively on HDL3 with respect to HDL2, and it is, for example, a polyoxyethylene polycyclic phenyl ether (for example, Newcol 610, Newcol 710 (manufactured by Nippon Nyukazai Co., Ltd.), ADEKA TOL PC-10, BLAUNON DSP-12.5, BLAUNON TSP-16 (manufactured by AOKI OIL INDUSTRIAL Co., Ltd.), or NOIGEN EA-137 (manufactured by DKS Co., Ltd.).

From the viewpoint of causing a surfactant to stably act on the lipoprotein C as a measurement target, the concentration of the surfactant in the second reagent composition is preferably 0.05% or more, more preferably 0.1% (w/v) or more, still and more preferably 0.5% (w/v) or more, and it is preferably 8.0% (w/v) or less and more preferably 5.0% (w/v) or less, with respect to the entire composition of the second reagent composition.

### (Protein having no enzymatic action, buffer solutions, and salt)

The kinds of the protein having no enzymatic action, the buffer solution, and the salt can be appropriately selected, for example, depending on the kind of enzyme contained in the second reagent composition. Examples of the specific configurations for these include those described above for the first reagent composition.

### (Coupler and hydrogen donor)

Examples of the specific configurations of the coupler and the hydrogen donors include those described above for the first reagent composition.

From the viewpoint of more stably measuring the lipoprotein C, the concentration of the hydrogen donor in the second reagent composition is preferably 1 mmol/L or more and more preferably 1.5 mmol/L or more, and it is preferably 10 mmol/L or less and more preferably 8 mmol/L or less.

From the viewpoint of more stably measuring the lipoprotein C, the concentration of the coupler in the second reagent composition is preferably 1 mM or more and more preferably 2 mM or more, and it is preferably 8 mM or less and more preferably 5 mM or less.

### (Iron complex)

Examples of the iron complex include potassium ferrocyanide, sodium ferrocyanide, a porphyrin iron complex, and an EDTA-iron complex.

The concentration of the iron complex in the second reagent composition is preferably 0.0015 mmol/L or more and preferably 0.3 mmol/L or less, and it may be, for example, 0.05 mmol/L or less, with respect to the entire composition of the second reagent composition.

### (Catalase inhibitor)

In a case where the first reagent composition has a catalase activity, the second reagent composition preferably contains a catalase inhibitor from the viewpoint of more stably measuring the lipoprotein C. Specific examples of the catalase inhibitor include sodium azide.

From the viewpoint of more stably measuring the lipoprotein C, the concentration of sodium azide in the second reagent composition is preferably 0.01% (w/v) or more and more preferably 0.02% (w/v) or more, and it is preferably 0.2% (w/v) or less and more preferably 0.1% (w/v) or less.

Next, the preferred configuration as a kit further includes the following kit.

From the viewpoint of more stably quantifying the lipoprotein C, it is preferable that in the kit, the first reagent composition contains one of a hydrogen donor and a coupler but does not contain the other thereof, and the second reagent composition does not contain the above one of the hydrogen donor and the coupler but contains the above other of thereof. That is, it is preferable that the coupler and hydrogen donor are respectively present separately in separate reagent compositions.

In addition, it is preferable that in the kit, the coupler and the hydrogen donor do not present together in the first or second reagent composition.

Further, it is preferable that in the kit, the first reagent composition satisfies one or two conditions of the following Conditions 1 to 3 and the second reagent composition does not satisfy the one or two conditions of Conditions 1 to 3, which the first reagent composition satisfy, but satisfies all conditions other than the one or two of Conditions 1 to 3:
Condition 1: containing a coupler;
Condition 2: containing an iron complex;
Condition 3: having peroxidase activity.

In the quantification of the lipoprotein C, for example, a hydrogen donor and a coupler are subjected to a coupling reaction in the presence of the peroxidase activity to generate a dye, which is used to measure the absorbance at the specific wavelength. At this time, an iron complex can be used as a reaction accelerating agent; however, in a case where the coupler, the peroxidase activity, and the iron complex are present in the same reagent, the reagent may gradually develop a color spontaneously, which affects the quantification of the lipoprotein C. In this regard, it is possible to suppress the spontaneous color development of the reagent composition by adopting the above configuration for the first and second reagent compositions as described above since it is possible to prevent the coexistence of the coupler, the peroxidase activity, and the iron complex in the first or second reagent composition. As a result, the quantification of the lipoprotein C can be carried out more stably.

From the viewpoint of more stably quantifying the lipoprotein C, it is more preferable that the first reagent composition satisfies Condition 1 and Condition 3 and the second reagent composition satisfies Condition 2.

### (Method)

The method of this embodiment is a method of quantifying lipoprotein C in a sample using the first and second reagent compositions described above, specifically a method of quantifying the lipoprotein C other than sdLDL. The quantification method in this embodiment includes the following first step and second step.
(First step): A step of acting the above-described first reagent composition on a sample
(Second step): A step of causing the above-described second reagent composition for quantifying the lipoprotein C as a measurement target to act, to quantify cholesterol in a remaining lipoprotein, after the first step

The configurations of the first and second reagent compositions are as described above.

In such a method, in a case of using the first and second reagent compositions in which absorbance ratio R1 satisfies the above conditions, the lipoprotein C as a measurement target can be stably quantified with high accuracy.

In addition, the quantification method for the lipoprotein C may be carried out, for example, by adding the first reagent composition to a sample (a test sample) to allow a reaction to proceed and then adding the second reagent composition thereto to allow a reaction to proceed, thereby measuring the absorbance.

The test sample is, for example, a blood-derived sample such as serum or plasma, where serum is preferable.

The first and second steps are usually carried out in an automated analysis device.

Examples of the automated analysis device include TBA-120FR and TBA-200FR (all, manufactured by TOSHIBA CORPORATION), JCA-BM1250, JCA-BM1650, and JCA-BM2250 (all, manufactured by JEOL Ltd.), HITACHI 7180 and HITACHI 7170 (all, manufactured by HITACHI, Ltd.), AU2700, AU5800, and AU680 (all, manufactured by OLYMPUS CORPORATION), and cobas c501 and cobas c701 (all, manufactured by Roche Diagnostics K.K.).

The amount of the sample and the amount of each reagent composition can be appropriately determined in consideration of, for example, the concentration of the reagent in each reagent composition, they are determined within the range applicable to the automated analysis device. For example, 1 to 10 µL of the test sample, 50 to 300 µL of the first reagent, and 25 to 200 µL of the second reagent may be used.

In this embodiment, regarding the reaction temperature, each step is preferably carried out at 2°C to 45°C and more preferably carried out at 25°C to 40°C.

Regarding the reaction time, each step is preferably carried out for 1 to 10 minutes and more preferably carried out for 3 to 7 minutes.

Each step will be described in more detail below.

### (First step)

In the first step, a sample is exposed to the first reagent composition. As a result, a lipoprotein other than the lipoprotein as a measurement target is erased, and cholesterol in the lipoprotein other than the lipoprotein as a measurement target is liberated and led out of the reaction system.

More specifically, in the first step, a surfactant that acts on a lipoprotein other than the lipoprotein as a measurement target is preferably allowed to act on a sample in the presence of at least one activity selected from the group consisting of a cholesterol esterase activity. Then, the cholesterol generated by the liberation from the lipoprotein is allowed to react with an enzyme such as cholesterol oxidase, which reacts with cholesterol, and led out of the reaction system. In the first step, it is possible to use a known technique by which cholesterol in a lipoprotein other than the lipoprotein as a measurement target is erased and led out of the reaction system, for example, aggregating cholesterol in the lipoprotein other than the lipoprotein as a measurement target or inhibiting it not to react in the subsequent step.

In the first step, the step of erasing cholesterol generated from a lipoprotein other than the lipoprotein as a measurement target and leading it out of the reaction system can be preferably carried out by any of the following methods.
(1) A method of generating hydrogen peroxide from cholesterol in a lipoprotein other than the lipoprotein as a measurement target by a cholesterol esterase activity and a cholesterol oxidase activity and degrading hydrogen peroxide into water and oxygen in the presence of a catalase activity
(2) A method of forming a colorless quinone in the presence of the hydrogen peroxide generated by the cholesterol esterase activity and the cholesterol oxidase activity, as well as a coupler or a hydrogen donor
(3) A method of degrading hydrogen peroxide in the presence of a catalase activity and simultaneously forming a colorless quinone in the presence of a coupler or hydrogen donor

In the method (1) or (2) above described, the cholesterol esterase and the cholesterol oxidase are allowed to act on cholesterol to generate hydrogen peroxide, and the generated hydrogen peroxide is erased.

In the above method, the first reagent composition has at least one activity selected from the group consisting of a cholesterol esterase activity and a cholesterol oxidase activity, and it has, for example, the following configuration.

In a case of the method (1) above described, the first reagent composition further has a catalase activity.

In a case of the method (2) above described, the first reagent composition contains at least one of a coupler and a hydrogen donor, and the first reagent composition further has a peroxidase activity.

In a case of the method (3) above described, the first reagent composition has a catalase activity and a peroxidase activity, and the first reagent composition contains a coupler or a hydrogen donor.

In the first step, the cholesterol in the lipoprotein not to be measured is led out of the reaction system, and thus in the subsequent step, it is possible to preferably cause the lipoprotein as a measurement target to remain selectively and more preferably to cause only the lipoprotein as a measurement target to remain, as a lipoprotein in the reaction solution. Eliminating a lipoprotein other than the lipoprotein as a measurement target in this way and leading it out of the reaction system so that the cholesterol in the lipoprotein other than the lipoprotein as a measurement target is not detected in the subsequent step may be referred to as "differentiating the lipoprotein as a measurement target from the other lipoprotein".

In addition, in the first step, the cholesterol in the lipoprotein other than the lipoprotein as a measurement target may not be completely erased. In that case, it is preferable to use such a surfactant that the lipoprotein C as a measurement target is selectively measured in the second step.

### (Second step)

In the second step, the lipoprotein C remaining after undergoing the first step is quantified. In the second step, it is possible to use a method of quantifying lipoprotein C, which is used in the related art, depending on the kind of lipoprotein.

Examples thereof include a method of quantifying the content of a specific aggregate formed by adding an aggregating agent according to turbidimetry, a method of using an antigen-antibody reaction with a specific antibody, a method of quantifying a degradation product using an enzyme. Among these, a method of quantifying a degradation product using an enzyme is preferable.

In the method of quantifying a degradation product using an enzyme, the second reagent composition containing, for example, one or two or more enzymes for measuring cholesterol, selected from the group consisting of a cholesterol esterase, a cholesterol oxidase, a cholesterol dehydrogenase, and a peroxidase is added to the reaction solution after the first step, to liberate and degrade lipoprotein C and quantifying the reaction product.

In addition, it is also preferable to use a second reagent composition containing a surfactant in the second step.

The quantification of the lipoprotein C is carried out, for example, by the absorbance measurement in a wavelength range of 580 to 720 nm, preferably 600 to 700 nm.

The embodiments of the present invention have been described above; however, these are examples of the present invention, and thus it is also possible to adopt various configurations other than those described above.

### [Example]

In each of the following examples, U-3900 manufactured by HITACHI Ltd. was used for the measurement of the absorption spectrum.

First, the components used in the reagent composition will be described.

In the following examples, the basic formulation of each reagent composition is as follows, depending on the kind of the lipoprotein C as a measurement target.

### (HDL-C)

### First reagent composition

| | |
|---|---|
| BES buffer solution, pH 7.0 | 100 mM |
| Cholesterol esterase | 800 U/L |
| Cholesterol oxidase | 1,400 U/L |
| Polyoxyethylene-polyoxypropylene block polymer | 0.1% |

### Second reagent composition

| | |
|---|---|
| BES buffer solution, pH 7.0 | 100 mM |
| Polyoxyethylene polycyclic phenyl ether | 1.0% |

### (LDL-C)

### First reagent composition

| | |
|---|---|
| PIPES buffer solution, pH 7.0 | 50 mM |
| Cholesterol esterase | 600 U/L |
| Cholesterol oxidase | 500 U/L |
| Polyoxyethylene polycyclic phenyl ether | 0.2% |
| BSA | 1.0% (w/v) |

### Second reagent composition

| | |
|---|---|
| PIPES buffer solution, pH 7.0 | 50 mM |
| Polyoxyethylene alkyl ether | 1.0% (w/v) |

### (HDL3-C)

### First reagent composition

| | |
|---|---|
| BES buffer solution, pH 7.0 | 100 mM |
| Cholesterol esterase | 2,000 U/L |
| Cholesterol oxidase | 300 U/L |
| Sphingomyelinase | 500 U/L |
| Polyoxyethylene-polyoxypropylene block polymer | 0.05% (w/v) |
| BSA | 1.0% (w/v) |

### Second reagent composition

| | |
|---|---|
| BES buffer solution, pH 7.0 | 100 mM |
| Polyoxyethylene polycyclic phenyl ether | 2.0% (w/v) |

### (Remnant-C)

### First reagent composition

| | |
|---|---|
| PIPES buffer solution, pH 6.8 | 50 mM |
| Cholesterol esterase (high molecular weight) | 5,000 U/L |
| Cholesterol oxidase | 2,500 U/L |
| Sphingomyelinase | 2,500 U/L |
| Polyoxyethylene polycyclic phenyl ether | 0.1% (w/v) |
| BSA | 1.0% (w/v) |

### Second reagent composition

| | |
|---|---|
| PIPES buffer solution, pH 6.8 | 50 mM |
| Cholesterol esterase (low molecular weight) | 4,000 U/L |
| Polyoxyethylene alkyl ether | 0.1% (w/v) |

### (apoE containing HDL-C)

### First reagent composition

| | |
|---|---|
| BES buffer solution, pH 7.0 | 100 mM |
| Cholesterol esterase | 800 U/L |
| Cholesterol oxidase | 1,600 U/L |
| Polyoxyethylene polycyclic phenyl ether | 0.067% (w/v) |

### Second reagent composition

| | |
|---|---|
| BES buffer solution, pH 7.0 | 100 mM |
| Polyoxyethylene polycyclic phenyl ether | 1.0% (w/v) |

### (Preparation Examples 1a to 11a)

For each example, in addition to the components described above, a first reagent composition containing the following components was prepared. That is, for respective examples, a total of five first reagent compositions respectively for LDL-C, HDL-C, HDL3-C, remnant-C, and apoE containing HDL-C were prepared.

### (Preparation Example 1a)

| | |
|---|---|
| Catalase (B-Cat) | 1,200 U/mL |
| 4-aminoantipyrine (4-AA) | 1.3 mM |

### (Preparation Example 2a)

| | |
|---|---|
| Peroxidase (POD) | 1.7U/mL |
| 4-aminoantipyrine | 1.3 mM |

### (Preparation Example 3a)

| | |
|---|---|
| 4-aminoantipyrine | 1.3 mM |
| Potassium ferrocyanide (FeK) | 0.036 mM |
| Catalase | 1,200 U/mL |

### (Preparation Example 4a)

| | |
|---|---|
| Peroxidase | 1.7U/mL |

### Hydrogen donor

| | |
|---|---|
| (HDAOS: HDL-C, apoE containing HDL-C) | 0.7 mM |
| (TOOS: LDL-C, HDL3-C, remnant-C) | 2.0 mM |

### (Preparation Example 5a)

### Hydrogen donor

| | |
|---|---|
| (HDAOS: HDL-C, apoE containing HDL-C) | 0.7 mM |
| (TOOS: LDL-C, HDL3-C, remnant-C) | 2.0 mM |
| Potassium ferrocyanide | 0.036 mM |
| Catalase | 1,200 U/mL |

### (Preparation Example 6a)

### Hydrogen donor

| | |
|---|---|
| (HDAOS: HDL-C, apoE containing HDL-C) | 0.7 mM |
| (TOOS: LDL-C, HDL3-C, remnant-C) | 2.0 mM |
| Potassium ferrocyanide | 0.036 mM |
| Peroxidase | 1.7U/mL |

### (Preparation Example 7a)

| | |
|---|---|
| 4-aminoantipyrine | 1.3 mM |
| Peroxidase | 1.7U/mL |
| Catalase | 1,200 U/mL |

### (Preparation Example 8a)

### Hydrogen donor

| | |
|---|---|
| (HDAOS: HDL-C, apoE containing HDL-C) | 0.7 mM |
| (TOOS: LDL-C, HDL3-C, remnant-C) | 2.0 mM |
| Peroxidase | 1.7U/mL |
| Catalase | 1,200 U/mL |

### (Preparation Example 9a)

### Hydrogen donor

| | |
|---|---|
| (HDAOS: HDL-C, apoE containing HDL-C) | 0.7 mM |
| (TOOS: LDL-C, HDL3-C, remnant-C) | 2.0 mM |
| Potassium ferrocyanide | 0.036 mM |
| Peroxidase | 1.7U/mL |
| Catalase | 1,200 U/mL |

### (Preparation Example 10a)

| | |
|---|---|
| Coupler (4-aminoantipyrine) | 1.3 mM |
| Peroxidase | 1.7U/mL |
| Potassium ferrocyanide | 0.036 mM |

### (Preparation Example 11a)

### Hydrogen donor

| | |
|---|---|
| (HDAOS: HDL-C, apoE containing HDL-C) | 0.7 mM |
| (TOOS: LDL-C, HDL3-C, remnant-C) | 2.0 mM |
| Catalase | 1,200 U/mL |

The obtained first reagent composition was subjected to the accelerated storage at 37°C for 1 week or 2 weeks, and the absorption spectrum at a wavelength of 320 nm to 480 nm was measured for spontaneous color development during storage. Table 1 shows the absorbance and absorbance ratio after the storage of the reagent composition obtained in each example.

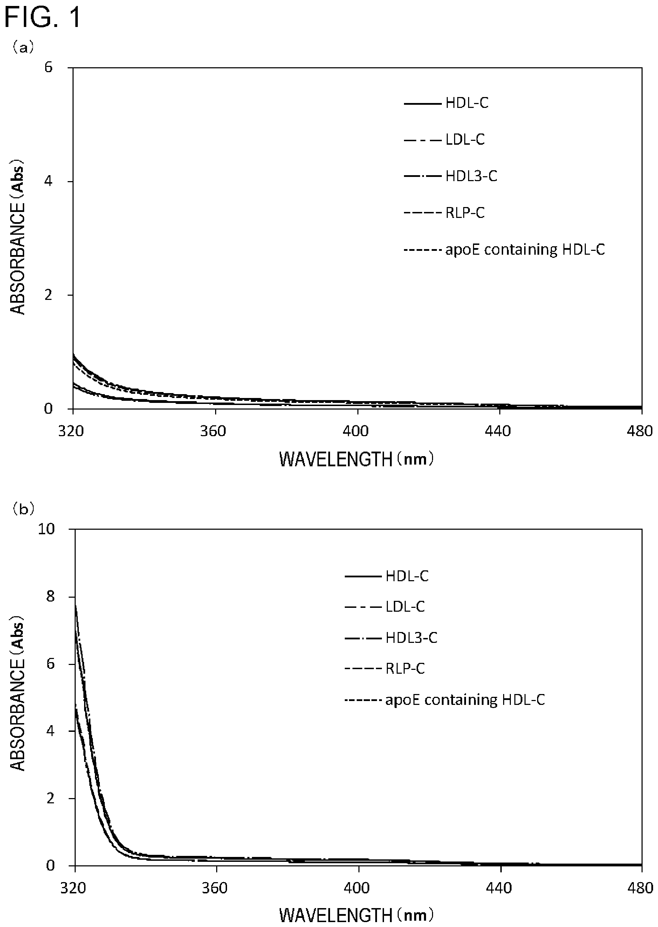

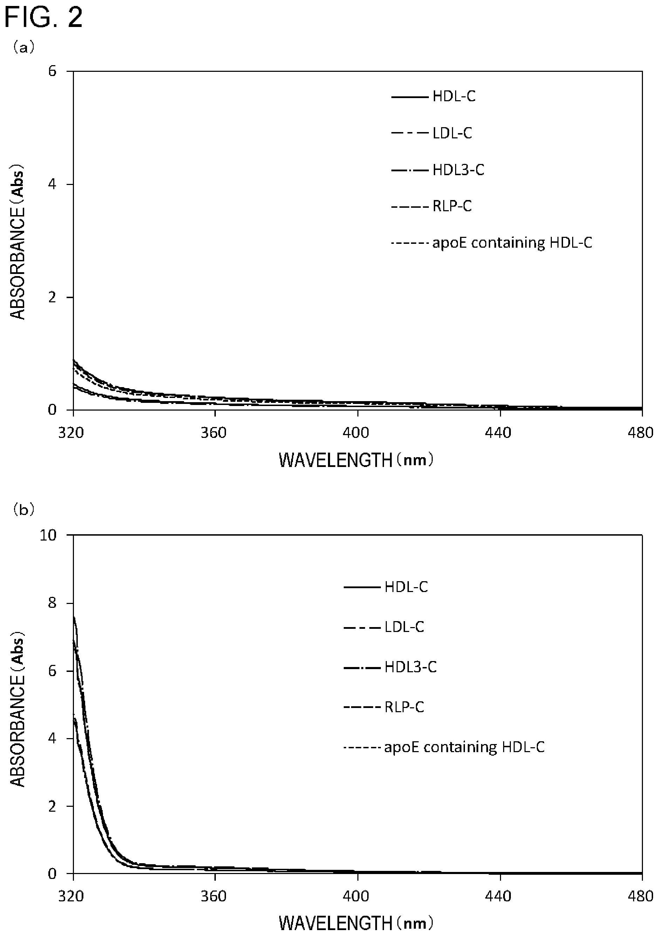

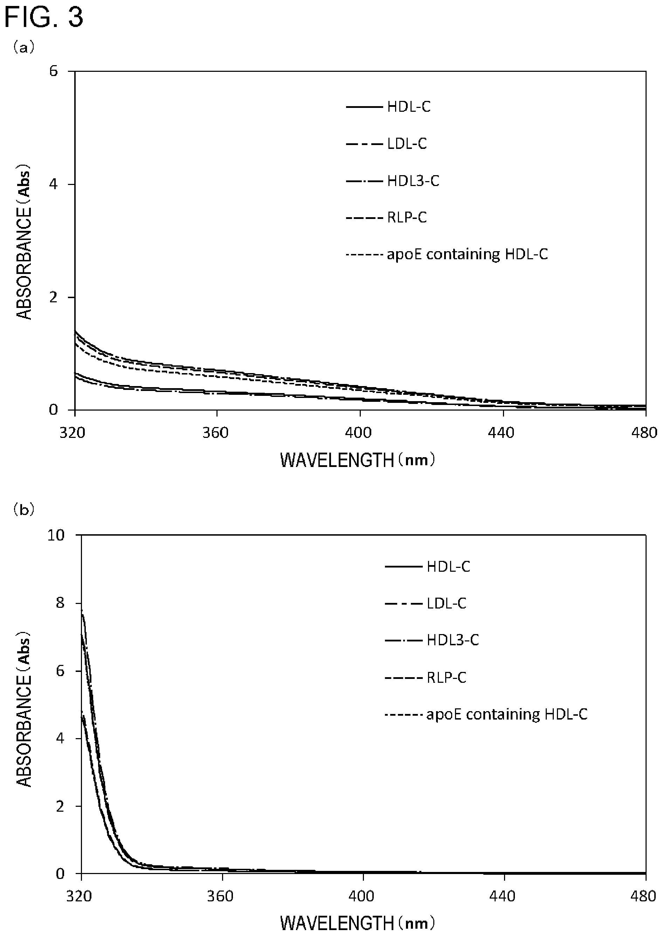

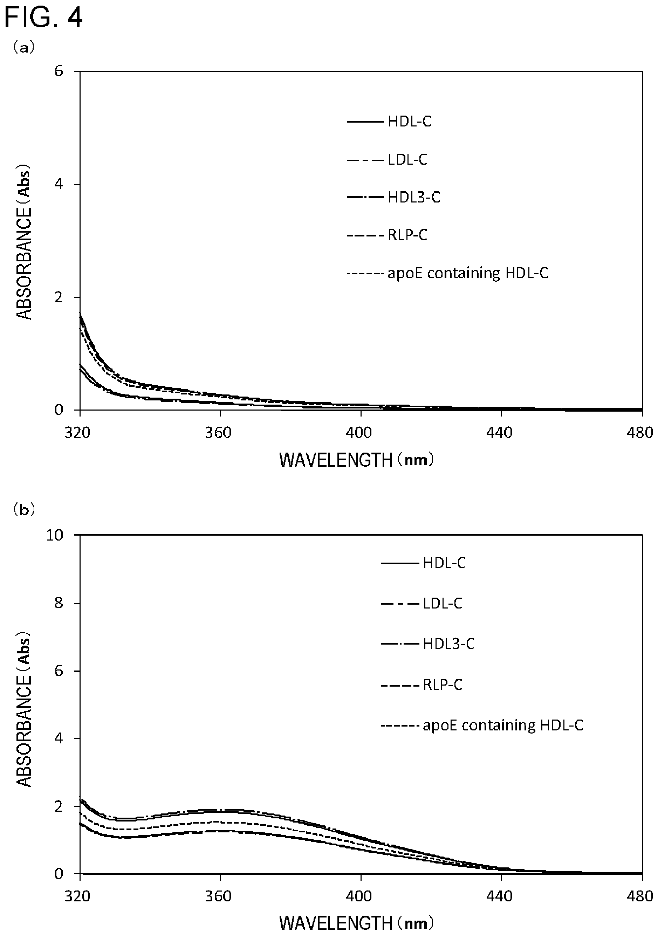

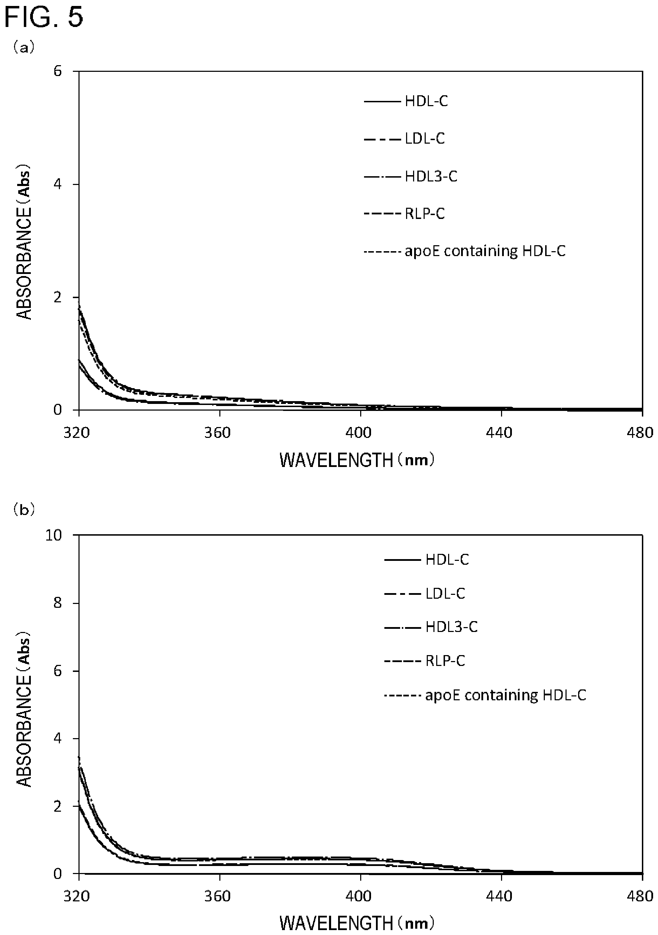

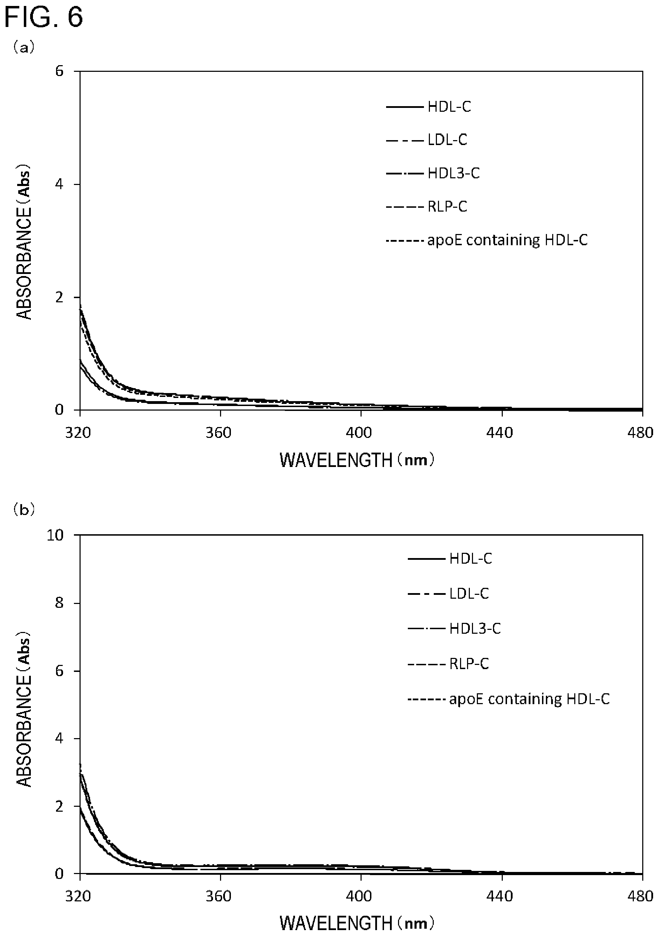

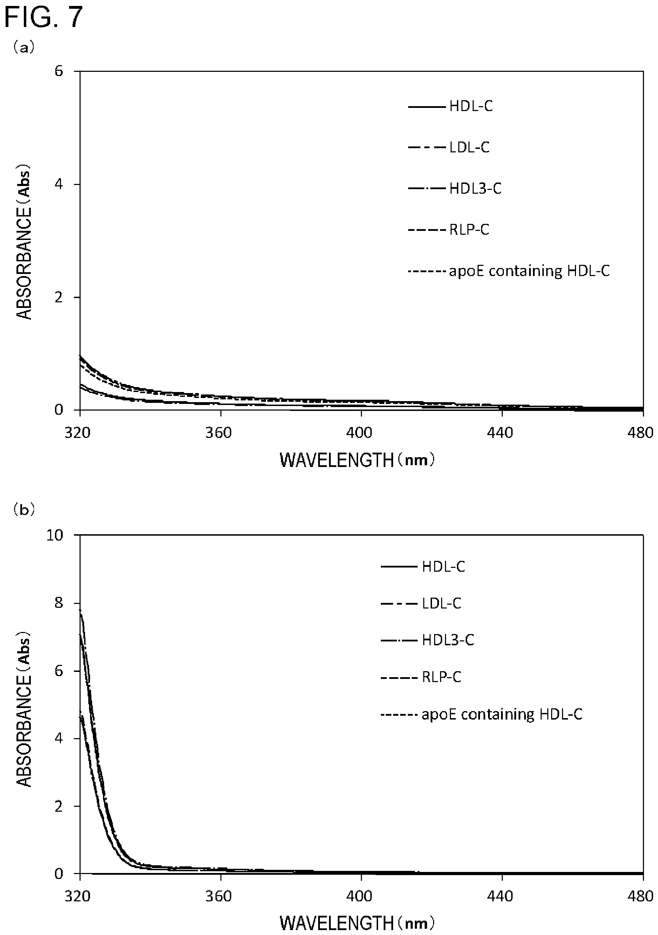

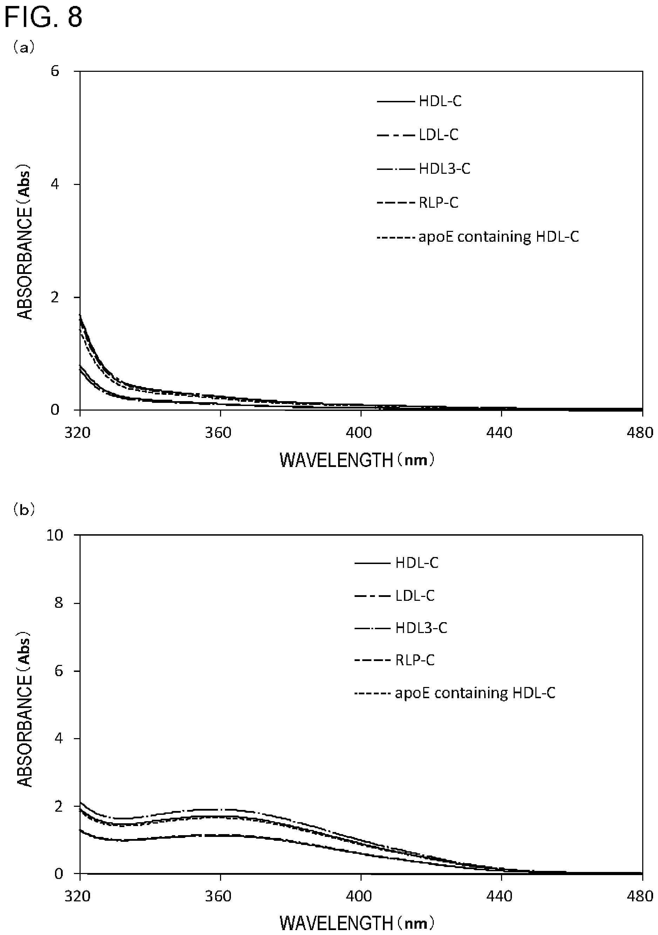

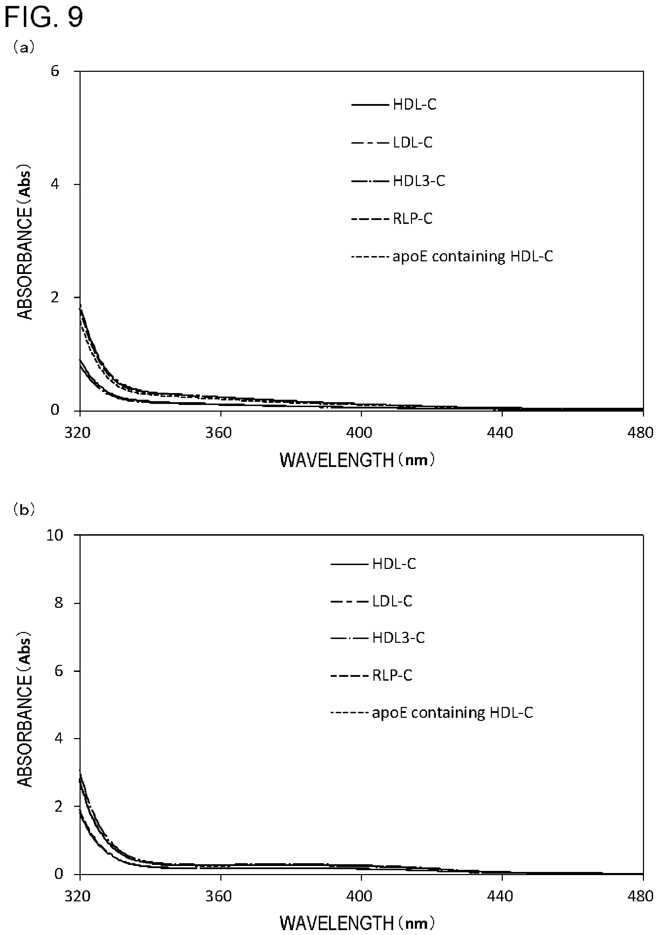

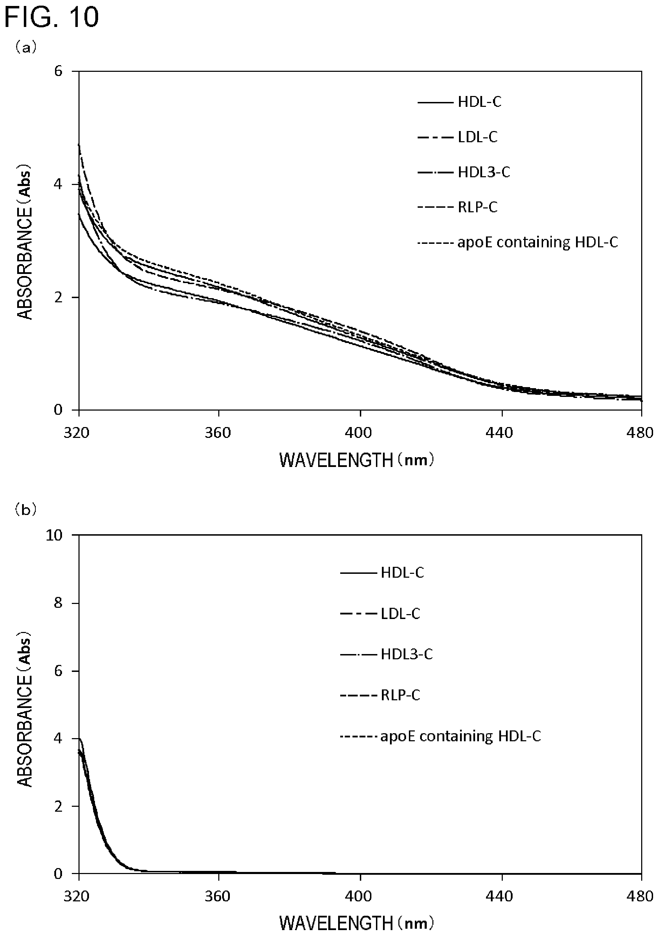

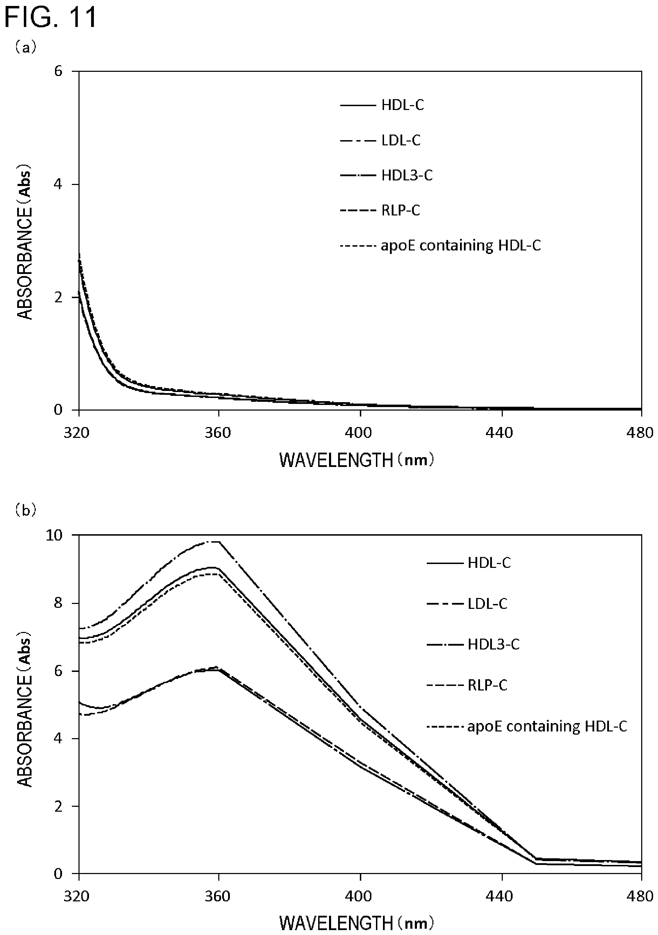

In addition, the respective sub-figures (a) in Fig. 1 to Fig. 11 are graphs showing absorption spectra of the first reagent compositions respectively obtained in Experimental Examples 1a to 11a after being subjected to the accelerated storage at 37°C for 2 weeks.

### [Table 1]

**Table 1**

| | | | Example 1-1 | Example 1-2 | Example 1-3 | Example 1-4 | Example 1-5 | Example 1-6 | Example 1-7 | Example 1-8 | Example 1-9 | Comparative Example 1-1 | Example 1-10 |
|---|---|---|---|---|---|---|---|---|---|---|---|---|---|
| | Preparation example | | Preparation example 1a | Preparation example 2a | Preparation example 3a | Preparation example 4a | Preparation example 5a | Preparation example 6a | Preparation example 7a | Preparation example 8a | Preparation example 9a | Preparation example 10a | Preparation example Ila |
| | Formula tion | TOOS or HDAOS | - | - | - | 2 mM | 2 mM | 2 mM | - | 2 mM | 2 mM | - | 2 mM |
| | | | | | | 0.7 mM | 0.7 mM | 0.7 mM | | 0.7 mM | 0.7 mM | | 0.7 mM |
| | | 4-AA | 1.3 mM | 1.3 mM | 1.3 mM | - | - | - | 1.3 mM | - | - | 1.3 mM | - |
| | | FeK | - | - | 0.036 Mm | - | 0.036 mM | 0.036 mM | - | - | 0.036 mM | 0.036 mM | - |
| | | POD | - | 1.7 KU/L | - | 1.7 KU/L | - | 1.7 KU/L | 1.7 KU/L | 1.7 KU/L | 1.7 KU/L | 1.7 KU/L | - |
| | | B-Cat | 1200 KU/L | - | 1200 KU/L | - | 1200 KU/L | - | 1200 KU/L | 1200 KU/L | 1200 KU/L | - | 1200 KU/L |
| | HDL-C | ABS450 | 0.029 | 0.035 | 0.057 | 0.018 | 0.016 | 0.016 | 0.035 | 0.017 | 0.017 | 0.318 | 0.037 |
| | | ABS400 | 0.059 | 0.076 | 0.198 | 0.047 | 0.045 | 0.049 | 0.078 | 0.046 | 0.053 | 1.134 | 0.105 |
| Reagent | | ABS360 | 0.099 | 0.116 | 0.336 | 0.130 | 0.105 | 0.105 | 0.116 | 0.113 | 0.108 | 1.931 | 0.271 |
| composition 1 | | R1 = ABS400/ABS450 | 2.07 | 2.17 | 3.48 | 2.62 | 2.78 | 3.00 | 2.23 | 2.76 | 3.14 | 3.57 | 2.85 |
| | | R2 = ABS360/ABS400 | 1.66 | 1.54 | 1.70 | 2.73 | 2.33 | 2.15 | 1.49 | 2.44 | 2.03 | 1.70 | 2.58 |
| | LDL-C | ABS450 | 0.061 | 0.066 | 0.120 | 0.038 | 0.034 | 0.035 | 0.074 | 0.036 | 0.036 | 0.358 | 0.031 |
| | | ABS400 | 0.125 | 0.144 | 0.415 | 0.101 | 0.096 | 0.103 | 0.164 | 0.097 | 0.113 | 1.279 | 0.085 |
| | | ABS360 | 0.206 | 0.222 | 0.709 | 0.274 | 0.222 | 0.222 | 0.244 | 0.239 | 0.228 | 2.177 | 0.217 |
| | | R1 = ABS400/ABS450 | 2.05 | 2.17 | 3.46 | 2.63 | 2.79 | 2.98 | 2.23 | 2.74 | 3.15 | 3.57 | 2.77 |
| | | R2 = ABS360/ABS400 | 1.65 | 1.53 | 1.71 | 2.72 | 2.32 | 2.15 | 1.48 | 2.45 | 2.03 | 1.70 | 2.55 |
| | HDL3-C | ABS450 | 0.025 | 0.031 | 0.050 | 0.016 | 0.014 | 0.014 | 0.031 | 0.015 | 0.015 | 0.289 | 0.031 |
| | | ABS400 | 0.053 | 0.067 | 0.176 | 0.042 | 0.040 | 0.044 | 0.069 | 0.041 | 0.048 | 1.226 | 0.085 |
| | | ABS360 | 0.087 | 0.103 | 0.298 | 0.115 | 0.094 | 0.093 | 0.103 | 0.100 | 0.096 | 1.898 | 0.217 |
| | | R1 = ABS400/ABS450 | 2.07 | 2.17 | 3.49 | 2.63 | 2.78 | 3.01 | 2.21 | 2.77 | 3.15 | 4.24 | 2.76 |
| | | R2 = ABS360/ABS400 | 1.66 | 1.54 | 1.69 | 2.73 | 2.33 | 2.14 | 1.49 | 2.44 | 2.02 | 1.55 | 2.55 |
| | RLP-C | ABS450 | 0.058 | 0.062 | 0.113 | 0.036 | 0.032 | 0.032 | 0.070 | 0.033 | 0.034 | 0.333 | 0.027 |
| | | ABS400 | 0.118 | 0.134 | 0.395 | 0.095 | 0.090 | 0.098 | 0.155 | 0.092 | 0.106 | 1.398 | 0.081 |
| | | ABS360 | 0.196 | 0.206 | 0.671 | 0.259 | 0.211 | 0.210 | 0.232 | 0.227 | 0.217 | 2.140 | 0.210 |
| | | R1 = ABS400/ABS450 | 2.04 | 2.18 | 3.48 | 2.62 | 2.78 | 3.02 | 2.23 | 2.76 | 3.13 | 4.20 | 2.95 |
| | | R2 = ABS360/ABS400 | 1.66 | 1.53 | 1.70 | 2.74 | 2.33 | 2.15 | 1.49 | 2.46 | 2.05 | 1.53 | 2.61 |
| | apoE contain | ABS450 | 0.052 | 0.054 | 0.101 | 0.032 | 0.029 | 0.029 | 0.062 | 0.030 | 0.030 | 0.370 | 0.038 |
| | | ABS400 | 0.105 | 0.118 | 0.353 | 0.084 | 0.080 | 0.087 | 0.138 | 0.082 | 0.095 | 1.320 | 0.110 |
| | ing HDL-C | ABS360 | 0.174 | 0.181 | 0.597 | 0.230 | 0.187 | 0.187 | 0.205 | 0.201 | 0.192 | 2.247 | 0.286 |
| | | R1 = ABS400/ABS450 | 2.03 | 2.17 | 3.49 | 2.62 | 2.77 | 3.00 | 2.23 | 2.76 | 3.14 | 3.57 | 2.92 |
| | | R2 = ABS360/ABS400 | 1.65 | 1.54 | 1.69 | 2.74 | 2.34 | 2.16 | 1.49 | 2.44 | 2.03 | 1.70 | 2.60 |

### (Preparation Examples 1b to 11b)

For each example, in addition to the components described above, a second reagent composition containing the following components was prepared. That is, for each example, a total of five second reagent compositions for LDL-C, HDL-C, HDL3-C, remnant-C, and apoE containing HDL-C, respectively, were prepared.

### (Preparation Example 1b)

### Hydrogen donor

| | |
|---|---|
| (HDAOS: HDL-C, apoE containing HDL-C) | 2.1 mM |
| (TOOS: LDL-C, HDL3-C, remnant-C) | 6.0 mM |
| Potassium ferrocyanide | 0.109 mM |
| Peroxidase | 5.0 U/mL |
| Sodium azide (NaN₃) | 0.05% (w/v) |

### (Preparation Example 2b)

### Hydrogen donor

| | |
|---|---|
| (HDAOS: HDL-C, apoE containing HDL-C) | 2.2 mM |
| (TOOS: LDL-C, HDL3-C, remnant-C) | 6.0 mM |
| Potassium ferrocyanide | 0.109 mM |

### (Preparation Example 3b)

### Hydrogen donor

| | |
|---|---|
| (HDAOS: HDL-C, apoE containing HDL-C) | 2.3 mM |
| (TOOS: LDL-C, HDL3-C, remnant-C) | 6.0 mM |
| Sodium azide | 0.05% (w/v) |
| Peroxidase | 5.0 U/mL |

### (Preparation Example 4b)

| | |
|---|---|
| 4-aminoantipyrine | 4.0 mM |
| Potassium ferrocyanide | 0.109 mM |

### (Preparation Example 5b)

| | |
|---|---|
| 4-aminoantipyrine | 4.0 mM |
| Peroxidase | 5.0 U/mL |
| Sodium azide | 0.05% (w/v) |

### (Preparation Example 6b)

| | |
|---|---|
| 4-aminoantipyrine | 4.0 mmol/L |

### (Preparation Example 7b)

### Hydrogen donor

| | |
|---|---|
| (HDAOS: HDL-C, apoE containing HDL-C) | 2.3 mM |
| (TOOS: LDL-C, HDL3-C, remnant-C) | 6.0 mM |
| Potassium ferrocyanide | 0.109 mM |
| Sodium azide | 0.05% (w/v) |

### (Preparation Example 8b)

| | |
|---|---|
| 4-aminoantipyrine | 4.0 mM |
| Potassium ferrocyanide | 0.109 mM |
| Sodium azide | 0.05% (w/v) |

### (Preparation Example 9b)

| | |
|---|---|
| 4-aminoantipyrine | 4.0 mM |
| Sodium azide | 0.05% (w/v) |

### (Preparation Example 10b)

### Hydrogen donor

| | |
|---|---|
| (HDAOS: HDL-C, apoE containing HDL-C) | 2.3 mM |
| (TOOS: LDL-C, HDL3-C, remnant-C) | 6.0 mM |

### (Preparation Example 11b)

| | |
|---|---|
| Coupler (4-aminoantipyrine) | 4.0 mM |
| Peroxidase | 5.0 U/mL |
| Potassium ferrocyanide | 0.109 mM |
| Sodium azide | 0.05% (w/v) |

The obtained second reagent composition was subjected to the accelerated storage at 37°C for 1 week or 2 weeks, and the absorption spectrum at a wavelength of 320 nm to 480 nm was measured for spontaneous color development during storage. Table 2 shows the absorbance and absorbance ratio after the storage of the reagent composition obtained in each example.

In addition, the respective sub-figures (b) in Fig. 1 to Fig. 11 are graphs showing absorption spectra of the second reagent compositions respectively obtained in Experimental Examples 1b to 11b after being subjected to the accelerated storage at 37°C for 2 weeks.

### [Table 2]

**Table 2**

| | | | Example 2-1 | Example 2-2 | Example 2-3 | Comparative Example 2-1 | Example 2-4 | Example 2-5 | Example 2-6 | Comparative Example 2-2 | Example 2-7 | Example 2-8 | Comparative Example 2-3 |
|---|---|---|---|---|---|---|---|---|---|---|---|---|---|
| | Preparation example | | Preparation example 1b | Preparation example 2b | Preparation example 3b | Preparation example 4b | Preparation example 5b | Preparation example 6b | Preparation example 7b | Preparation example 8b | Preparation example 9b | Preparation example 11b | Preparation example 11b |
| | Formulation | TOOS or HDAOS | 6 mM | 6 mM | 6 mM | - | - | - | 6 mM | | - | 6 mM | - |
| | | | 2.1 mM | 2.2 mM | 2.3 mM | | | | 2.3 mM | | | 2.3 mM | |
| | | 4-AA | - | - | - | 4 mM | 4 mM | 4 mM | - | 4 mM | 4 mM | - | 4 mM |
| | | FeK | 0.109 mM | 0.109 mM | - | 0.109 mM | - | - | 0.109 mM | 0.109 mM | - | - | 0.109 mM |
| | | POD | 5 KU/L | - | 5 KU/L | - | 5 KU/L | - | - | - | - | - | 5 KU/L |
| | | NaN₃ | 0.05% | - | 0.05% | - | 0.05% | - | 0.05% | 0.05% | 0.05% | - | 0.05% |
| | HDL-C | ABS450 | 0.059 | 0.041 | 0.040 | 0.103 | 0.067 | 0.041 | 0.040 | 0.087 | 0.044 | 0.008 | 0.462 |
| | | ABS400 | 0.177 | 0.086 | 0.070 | 1.055 | 0.429 | 0.224 | 0.070 | 0.906 | 0.241 | 0.026 | 4.583 |
| | | ABS360 | 0.231 | 0.188 | 0.150 | 1.847 | 0.426 | 0.235 | 0.150 | 1.733 | 0.261 | 0.068 | 9.022 |
| | | R1 = ABS400/ABS450 | 3.03 | 2.10 | 1.74 | 10.22 | 6.38 | 5.40 | 1.74 | 10.42 | 5.47 | 3.29 | 9.92 |
| | | R2 = ABS360/ABS400 | 1.30 | 2.20 | 2.16 | 1.75 | 0.99 | 1.05 | 2.16 | 1.91 | 1.08 | 2.61 | 1.97 |
| | LDL-C | ABS450 | 0.039 | 0.027 | 0.027 | 0.073 | 0.044 | 0.028 | 0.027 | 0.056 | 0.029 | 0.008 | 0.295 |
| Reagent | | ABS400 | 0.117 | 0.056 | 0.046 | 0.733 | 0.281 | 0.147 | 0.046 | 0.609 | 0.159 | 0.024 | 3.180 |
| composition 2 | | ABS360 | 0.153 | 0.124 | 0.099 | 1.291 | 0.280 | 0.155 | 0.099 | 1.156 | 0.171 | 0.061 | 6.033 |
| | | R1 = ABS400/ABS450 | 3.03 | 2.09 | 1.73 | 10.02 | 6.35 | 5.34 | 1.72 | 10.95 | 5.50 | 3.13 | 10.79 |
| | | R2 = ABS360/ABS400 | 1.31 | 2.21 | 2.16 | 1.76 | 1.00 | 1.05 | 2.16 | 1.90 | 1.08 | 2.57 | 1.90 |
| | HDL3-C | ABS450 | 0.065 | 0.045 | 0.044 | 0.107 | 0.074 | 0.046 | 0.044 | 0.092 | 0.049 | 0.006 | 0.437 |
| | | ABS400 | 0.196 | 0.094 | 0.077 | 1.108 | 0.474 | 0.246 | 0.077 | 1.016 | 0.268 | 0.022 | 4.930 |
| | | ABS360 | 0.255 | 0.208 | 0.166 | 1.923 | 0.471 | 0.260 | 0.167 | 1.926 | 0.288 | 0.061 | 9.825 |
| | | R1 = ABS400/ABS450 | 3.01 | 2.10 | 1.74 | 10.31 | 6.37 | 5.38 | 1.75 | 11.00 | 5.46 | 3.87 | 11.29 |
| | | R2 = ABS360/ABS400 | 1.30 | 2.21 | 2.16 | 1.74 | 0.99 | 1.06 | 2.17 | 1.89 | 1.08 | 2.71 | 1.99 |
| | RLP-C | ABS450 | 0.040 | 0.028 | 0.028 | 0.071 | 0.046 | 0.029 | 0.028 | 0.057 | 0.030 | 0.009 | 0.293 |
| | | ABS400 | 0.121 | 0.059 | 0.048 | 0.718 | 0.294 | 0.153 | 0.048 | 0.622 | 0.166 | 0.025 | 3.288 |
| | | ABS360 | 0.159 | 0.129 | 0.103 | 1.265 | 0.292 | 0.161 | 0.104 | 1.183 | 0.178 | 0.062 | 6.095 |
| | | R1 = ABS400/ABS450 | 3.02 | 2.11 | 1.72 | 10.16 | 6.38 | 5.34 | 1.73 | 10.92 | 5.51 | 2.88 | 11.21 |
| | | R2 = ABS360/ABS400 | 1.31 | 2.20 | 2.17 | 1.76 | 0.99 | 1.05 | 2.18 | 1.90 | 1.08 | 2.51 | 1.85 |
| | apoE containing | ABS450 | 0.058 | 0.040 | 0.039 | 0.086 | 0.067 | 0.040 | 0.040 | 0.083 | 0.043 | 0.008 | 0.453 |
| | | ABS400 | 0.174 | 0.084 | 0.069 | 0.862 | 0.424 | 0.220 | 0.069 | 0.871 | 0.234 | 0.024 | 4.491 |
| | HDL-C | ABS360 | 0.228 | 0.186 | 0.149 | 1.514 | 0.422 | 0.232 | 0.147 | 1.678 | 0.257 | 0.061 | 8.841 |
| | | R1 = ABS400/ABS450 | 3.03 | 2.10 | 1.74 | 10.07 | 6.31 | 5.46 | 1.72 | 10.46 | 5.42 | 3.12 | 9.92 |
| | | R2 = ABS360/ABS400 | 1.31 | 2.21 | 2.17 | 1.76 | 1.00 | 1.05 | 2.14 | 1.93 | 1.10 | 2.57 | 1.97 |

### (Examples 3-1 to 3-7 and Comparative Examples 3-1 to 3-4)

Using LDL-C as a representative item, the first and second reagent compositions in each example were stored under the conditions of 37°C for 2 weeks and subjected to an accelerated test. The first and second reagent compositions after storage were used in the combinations shown in Table 3 to evaluate the accuracy of the lipoprotein C measurement. The evaluation results are collectively shown in Table 3.

In each measurement system, a calibration curve was created by using a standard substance having a known LDL-C concentration and a physiological saline solution as a blank substance containing no LDL-C. Then, a low-value sample was subjected to the measurement to measure the minimum detection limit.

Specifically, for each example shown in Table 3, the LDL-C concentration was evaluated according to the following procedure.

### (Method of measuring and evaluating LDL-C concentration)

1. 150 µL of the first reagent composition of each example was added to 2.0 µL of a sample of the standard substance and a physiological saline solution and allowed to act thereon at 37°C for 5 minutes.
2. 50 µL of the second reagent composition of each example was added thereto and allowed to act thereon at 37°C for 5 minutes.
3. Immediately before adding the second reagent composition and 5 minutes after adding the second reagent composition, the absorbance at a wavelength of 600 nm (a main wavelength) and 700 nm (a minor wavelength) was measured to calculate the calibration absorbance from (the absorbance at 600 nm 5 minutes after the addition of the second reagent composition - the absorbance at 700 nm) - (the absorbance at 600 nm immediately before the addition of the second reagent composition - the absorbance at 700 nm), thereby creating a calibration curve.
4. Next, a low-concentration (4.0 mg/dL) sample having a known LDL-C value was diluted with a physiological saline solution to prepare 10-step dilution series samples, and the physiological saline solution and each dilution series were each subjected to quintuple measurement with the above reagents.
5. The accuracy of the measurement was evaluated as "o" in a case where the detection limit is 2.0 mg/dL or lower and as "×" in a case where it is higher than 2.0 mg/dL, where the detection limit is set as the lowest concentration that satisfies "the average value of the physiological saline solution + 2.6 SD < the average value of diluted sample - 2.6 SD".

### [Table 3]

**Table 3**

| | Example 3-1 | Example 3-2 | Example 3-3 | Comparative Example 3-1 | Example 3-4 | Example 3-5 | Example 3-6 | Comparative Example 3-2 | Example 3-7 | Comparative Example 3-3 | Comparative Example 3-4 |
|---|---|---|---|---|---|---|---|---|---|---|---|
| First reagent composition | Example 1-1 | Example 1-2 | Example 1-3 | Example 1-4 | Example 1-5 | Example 1-6 | Example 1-7 | Example 1-8 | Example 1-9 | Comparative Example 1-1 | Example 1-10 |
| Second reagent composition | Example 2-1 | Example 2-2 | Example 2-3 | Comparative Example 2-1 | Example 2-4 | Example 2-5 | Example 2-6 | Comparative Example 2-2 | Example 2-7 | Example 2-8 | Comparative Example 2-3 |
| Detection limit (mg/dL) | 1.2 | 0.8 | 2 | 2.8 | 0.8 | 1.2 | 0.8 | 2.8 | 1.6 | 2.4 | 3.2 |
| Evaluation | ○ | ○ | ○ | × | ○ | ○ | ○ | × | ○ | × | × |

In Examples where both R1 and R2 of the first and second reagent compositions were respectively within the specific ranges, LDL-C could be detected accurately.

This application claims priority based on Japanese Patent Application No. 2020-096095 filed on June 2, 2020, and all contents of the disclosure are incorporated herein.

## Claims

1. A kit that is used for fractionation of cholesterol (lipoprotein C) in a lipoprotein other than small dense LDL in a sample, the kit comprising:
a first reagent composition having at least one activity selected from the group consisting of a cholesterol esterase activity and a cholesterol oxidase activity; and
a second reagent composition for quantifying the lipoprotein C as a measurement target,
wherein in an absorption spectrum after storing the first reagent composition at 37°C for two weeks, a ratio R1 represented by ABS400/ABS450 is 0.90 or more and 3.50 or less in a case where an absorbance at a wavelength of 400 nm is denoted by ABS400 and an absorbance at a wavelength of 450 nm is denoted by ABS450, and
in an absorption spectrum after storing the second reagent composition at 37°C for two weeks, a ratio R1 represented by ABS400/ABS450 is 0.90 or more and 9.00 or less in a case where an absorbance at a wavelength of 400 nm is denoted by ABS400 and an absorbance at a wavelength of 450 nm is denoted by ABS450.

2. The kit according to claim 1,
wherein in the absorption spectrum after storing the first reagent composition at 37°C for 2 weeks, a ratio R2 represented by ABS360/ABS400 is 0.90 or more and 3.00 or less in a case where an absorbance at a wavelength of 360 nm is denoted by ABS360.

3. The kit according to claim 1 or 2,
wherein the first reagent composition has at least one activity selected from the group consisting of a peroxidase activity and a catalase activity.

4. The kit according to any one of claims 1 to 3,
wherein the first reagent composition contains a surfactant that acts on a lipoprotein other than the lipoprotein as a measurement target.

5. The kit according to any one of claims 1 to 4,
wherein in the absorption spectrum after storing the second reagent composition at 37°C for 2 weeks, a ratio R2 represented by ABS360/ABS400 is 0.90 or more and 2.50 or less in a case where an absorbance at a wavelength of 360 nm is denoted by ABS360.

6. The kit according to any one of claims 1 to 5,
wherein the second reagent composition contains a surfactant that acts on the lipoprotein as a measurement target.

7. The kit according to any one of claims 1 to 6,
wherein the first reagent composition satisfies one or two of the following Conditions 1 to 3, and
the second reagent composition does not satisfy the one or two of Conditions 1 to 3 but satisfies all conditions other than the one or two of Conditions 1 to 3:
Condition 1: containing a coupler;
Condition 2: containing an iron complex; and
Condition 3: having peroxidase activity.

8. The kit according to any one of claims 1 to 7,
wherein the lipoprotein C as a measurement target is LDL cholesterol, HDL cholesterol, HDL3 cholesterol, remnant cholesterol, or apoE containing HDL cholesterol.

9. A method of quantifying cholesterol (lipoprotein C) in a lipoprotein other than small dense LDL in a sample, the method comprising:
causing a first reagent composition having at least one activity selected from the group consisting of a cholesterol esterase activity and a cholesterol oxidase activity, to act on the sample; and
after the causing the first reagent composition to act on the sample, causing the second reagent composition for quantifying the lipoprotein C as a measurement target to act, to quantify cholesterol in a remaining lipoprotein,
wherein in an absorption spectrum after storing the first reagent composition at 37°C for two weeks, a ratio R1 represented by ABS400/ABS450 is 0.90 or more and 3.50 or less in a case where an absorbance at a wavelength of 400 nm is denoted by ABS400 and an absorbance at a wavelength of 450 nm is denoted by ABS450, and
in an absorption spectrum after storing the second reagent composition at 37°C for two weeks, a ratio R1 represented by ABS400/ABS450 is 0.90 or more and 9.00 or less in a case where an absorbance at a wavelength of 400 nm is denoted by ABS400 and an absorbance at a wavelength of 450 nm is denoted by ABS450.

10. The method according to claim 9,
wherein in the absorption spectrum after storing the first reagent composition at 37°C for 2 weeks, a ratio R2 represented by ABS360/ABS400 is 0.90 or more and 3.00 or less in a case where an absorbance at a wavelength of 360 nm is denoted by ABS360.

11. The method according to claim 9 or 10,
wherein the first reagent composition further has at least one activity selected from the group consisting of a peroxidase activity and a catalase activity.

12. The method according to any one of claims 9 to 11,
wherein the first reagent composition contains a surfactant that acts on a lipoprotein other than the lipoprotein as a measurement target.

13. The method according to any one of claims 9 to 12,
wherein in the absorption spectrum after storing the second reagent composition at 37°C for 2 weeks, a ratio R2 represented by ABS360/ABS400 is 0.90 or more and 2.50 or less in a case where an absorbance at a wavelength of 360 nm is denoted by ABS360.

14. The method according to any one of claims 9 to 13,
wherein the second reagent composition contains a surfactant that acts on the lipoprotein as a measurement target.

15. The method according to any one of claims 9 to 14,
wherein the first reagent composition satisfies one or two of the following Conditions 1 to 3, and
the second reagent composition does not satisfy the one or two of Conditions 1 to 3 but satisfies all conditions other than the one or two of Conditions 1 to 3:
Condition 1: containing a coupler;
Condition 2: containing an iron complex; and
Condition 3: having peroxidase activity.

16. The method according to any one of claims 9 to 15,
wherein the lipoprotein C as a measurement target is LDL cholesterol, HDL cholesterol, HDL3 cholesterol, remnant cholesterol, or apoE containing HDL cholesterol.
